# EUROPEAN PATENT APPLICATION

(11) **EP 4 471 793 A1**
(43) Date of publication of application: **04.12.2024**
(21) Application number: 24177915.6
(22) Date of filing: 24.05.2024
(51) Int. Cl.: G16H 40/20, G16H 50/20, G16H 50/80, G08B 21/24

(54) **HYGIENIC EARLY-STAGE WARNING SYSTEM**

(30) Priority: 31.05.2023 US 202363505182 P
(71) Applicant: OP-Hygiene IP GmbH, 4704 Niederbipp (CH); University of Freiburg, 79098 Freiburg (DE)
(72) Inventor: Ophardt, Heiner, 4422 Arisdorf (CH); Claudinon, Julie, 79108 Freiburg (DE); Römer, Winfried, 79189 Bad Krozingen (DE); Lang, Albrecht, 4704 Niederbipp (CH); Madec, Morgan, F-67100 Strasbourg (FR); Disegna, Sofia Catharina, 79117 Freiburg (DE); Steltenkamp, Siegfried, 53229 Bonn (DE); Perronno, Paul, 67000 Strasbourg (FR)
(74) Representative: Grünecker Patent- und Rechtsanwälte PartG mbB

(57) **Abstract**

A system including a plurality of fluid dispensers for dispensing hand cleaning fluid. At least some of the fluid dispensers are configured to analyze at least some of the hand cleaning fluid after the hand cleaning fluid has contacted a user's hand. The system is configured to detect and/or predict an infection based at least in part on the analysis of the hand cleaning fluid. The system is preferably capable of predicting the spatial and temporal distribution of an infection.

## Description

### Related Application

This application claims priority to the 31 May 2023 filing date of United States Provisional Patent Application Serial No. 63/505,182, which is incorporated herein by reference.

### Field of the Invention

This invention relates to a system for detecting an infection, and more particularly to a system that is configured to detect and/or predict the spread of an infection based on an analysis of hand cleaning fluid dispensed onto a user's hand.

### Background of the Invention

Many human and animal diseases are caused by infectious agents such as viruses, bacteria, fungi, prions, and parasites. In order to limit the spread of these diseases, it is often useful to identify infected individuals, so that precautions can be taken to limit their risk of transmitting the infection to others. For example, once an infected person is identified, they may be able to seek treatment for the disease, which may for example reduce the amount of time that they remain infectious. They may also be able to engage in practices such as social distancing to reduce their risk of transmitting the disease to others.

The applicant has appreciated a number of limitations and disadvantages of prior art methods of identifying infected individuals. For example, many tests for infectious diseases are expensive, complex, invasive and/or time intensive. These disadvantages of prior art methods can significantly reduce their capacity to rapidly identify infected individuals.

A further limitation of the prior art is that typically individuals are only tested for a disease after they have developed symptoms. As many infectious diseases are contagious before the onset of symptoms, an infected individual may be infectious for a significant period of time before they are ultimately tested, during which time they may spread the disease to others. In some cases, contagious individuals may remain asymptomatic or may only develop mild symptoms, and thus never get tested. In many cases, the available test is too expensive, complex, and/or labor or time intensive for widespread testing to be offered to asymptomatic individuals.

An additional limitation of the prior art is that tests are typically designed to detect a specific, previously known disease or disease causing agent. As such, in order to screen individuals for a variety of different possible infections, several different tests would need to be administered, each of which may be expensive, complex, time intensive, resource intensive, and/or labor intensive. For this reason, widespread screening of a large population for a wide variety of different possible infections may not be feasible. Furthermore, many existing tests may be unable to detect novel diseases. This inability to test for novel diseases may prevent public health authorities from rapidly recognizing when a new disease is present in a population, and may hinder efforts to contain the spread of the disease.

### Summary of the Invention

To at least partially overcome some of the disadvantages of previously known systems, methods, and devices, in one aspect the present invention provides a system comprising a plurality of fluid dispensers for dispensing hand cleaning fluid, wherein at least some of the fluid dispensers are configured to analyze at least some of the hand cleaning fluid after the hand cleaning fluid has contacted a user's hand. The system is preferably configured to detect an infection based at least in part on the analysis of the hand cleaning fluid.

The inventors of the present invention have appreciated that a system of hand cleaning fluid dispensers can advantageously be used to screen a large number of individuals for possible infections, including individuals who are asymptomatic or who might not otherwise seek out testing. This preferably allows the system to detect infections early, so that appropriate actions can be taken to limit the spread of the infections.

In preferred embodiments, the system includes fluid dispensers that are dispersed at different locations in an environment, such as a hospital, a building, a city, or a country. The system is preferably able to determine where in the environment an infection is present, based on the location of the fluid dispenser or dispensers where the infection was detected. This preferably allows precautionary measures to be appropriately focused on the specific location where the infection is present, for example by requiring mask wearing, enhanced hand hygiene, enhanced screening, or limited travel to and/or from the affected area or zone.

The system is also preferably able to trace the path that an infection has spread through the environment over time, based on infection detection data collected by the system over time. For example, the system may detect an infection in a first zone of the environment on day 0 of an outbreak; in the first zone and a second zone on day 1 of the outbreak; and in the first zone, the second zone, and a third zone on day 2 of the outbreak. Based on the collected data, the system may be able to determine that the infection spread from zone 1 to zone 2 in approximately one day, and from zone 2 to zone 3 in approximately one day. This data may be helpful for determining the appropriate public health measures that may be taken to limit the spread of the infection.

The system is also preferably able to predict the future spread of an infection over time. The system may, for example, include a machine learning algorithm that is able to make predictions based on data that is collected by the system and/or data that is provided to the system from an external source. For example, the system may record the paths that various infections spread through the environment over time, and then use this information to predict the spread of future infections. The system may also receive information from external sources, such as traffic data or transit data that indicates how people are moving through the environment, and use this data to predict how an infection might spread through the environment. The prediction can then preferably be used to take appropriate public health measures to limit the spread of the infection, for example by requiring mask wearing, enhanced hand hygiene, enhanced screening, or limited travel to and/or from the zone or zones where it is predicted that the infection will spread.

In some embodiments of the invention, the system is configured to inactivate a fluid analysis mechanism of at least some of the fluid dispensers when certain conditions are met, such as when there are no detected infections in a zone or environment. This preferably helps to reduce waste and increase the length of time that the fluid dispensers can operate before requiring service or maintenance. Preferably the system includes one or more sentinel fluid dispensers in which the fluid analysis mechanisms remain active. Preferably there is always at least one sentinel fluid dispenser with an active fluid analysis mechanism in each zone of the environment.

Preferably, the system is configured to modify an operation of the system when one of the sentinel fluid dispensers detects an infection. For example, the system may be configured to activate the fluid analysis mechanisms of some or all of the fluid dispensers in the zone where the infection was detected, so that the system is able to collect additional data about the prevalence of the infection in that zone. The system may also be configured to activate some or all of the fluid analysis mechanisms of the fluid dispensers in one or more of the other zones in the environment, such as in the zone or zones where the system predicts the infection will spread to in the future.

The inventors have appreciated that the fluid analysis mechanisms may use a variety of different techniques for analyzing the hand cleaning fluid, such as electrical, acoustic, optical, magnetic, spectroscopic, electromagnetic, mechanical, thermal, and/or chemical methods. In one preferred embodiment, the fluid analysis mechanism includes an analysis chamber for receiving at least some of the hand cleaning fluid after the hand cleaning fluid has contacted a user's hand, and a camera for capturing at least one image of the hand cleaning fluid received by the analysis chamber.

The system is preferably able to analyze the at least one image to determine whether there is evidence of a possible infection. For example, in one preferred embodiment the system is configured to identify particles present in the at least one image, and to categorize the particles into different classes. The classes preferably include at least one recognized class and at least one unrecognized class.

The system is preferably configured to categorize the particles that meet predetermined criteria into the at least one recognized class, and to categorize the particles that fail to meet the predetermined criteria into the at least one unrecognized class. The predetermined criteria may include characteristics of the particles such as size, shape, and movement, including movement in three dimensions, including three dimensional momentum and three dimensional acceleration.

The system is preferably configured to compare the quantity of particles categorized into each of the classes with a background rate. The background rate may, for example, be determined by the system based on training data collected by the fluid dispensers over time. If the system determines that the quantity of particles in one or more of the classes exceeds the background rate, the system may be configured to determine that an infection has been detected. In at least some embodiments, the system will only determine that an infection is present if the quantity of particles in one or more of the classes exceeds the background rate by a threshold amount. The threshold amount may be selected by an administrator of the system, or can be set and/or adjusted by the system itself using a machine learning algorithm to improve performance over time.

The inventors have appreciated that by allowing the system to categorize particles into at least one unrecognized class, the system is preferably able to detect and quantify the presence of new infectious particles that have not previously been encountered by the system. This preferably allows the system to rapidly detect possible new infectious agents spreading through a population, so that immediate actions can be taken to limit the spread.

In some preferred embodiments of the invention, the fluid analysis mechanism is configured to perform a stopped-flow analysis of the hand cleaning fluid. In the stopped-flow analysis, the fluid is pumped into the analysis chamber and the flow of the fluid is then stopped. The camera then takes a series of images of the fluid in the analysis chamber over time, so that the movement of the particles over time can be detected.

The system is preferably configured to determine whether a particle is active or inactive based on the movement of the particle over time. For example, a dead or inactive particle would normally drift a short distance in the direction of the previous flow, due to the momentum of the particle. In contrast, an active and alive particle may exhibit a pattern of movement akin to a random-walk, with for example the flagella of a live bacteria propelling the bacteria through the fluid in different directions.

The system is preferably able to distinguish between dead or inactive particles and live or active particles, based at least in part on the movement of the particles over time. The system may, for example, be configured to determine whether a hand sanitizing event was successful or not based on the presence or absence of live bacteria in the analyzed fluid. If a hand sanitizing event was unsuccessful, the system may be configured to prompt the user to engage in further hand cleaning, and/or may alter a formulation of the hand cleaning fluid.

Further aspects of the invention include the following:
1. A system comprising: a plurality of fluid dispensers for dispensing hand cleaning fluid; wherein at least some of the fluid dispensers are configured to analyze at least some of the hand cleaning fluid after the hand cleaning fluid has contacted a user's hand; and wherein the system is configured to detect an infection based at least in part on the analysis of the hand cleaning fluid.
2. A system, which optionally includes one or more features of any one or more of the previous aspects, wherein the plurality of fluid dispensers are located at a plurality of different locations within an environment.
3. A system, which optionally includes one or more features of any one or more of the previous aspects, wherein the environment has a plurality of different zones; wherein at least one of the fluid dispensers is located in each of the zones; and wherein, when the system detects an infection, the system is configured to identify which of the zones the infection is located in based on the location of the fluid dispenser where the infection was detected.
4. A system, which optionally includes one or more features of any one or more of the previous aspects, wherein the environment comprises a building.
5. A system, which optionally includes one or more features of any one or more of the previous aspects, wherein the environment comprises a hospital.
6. A system, which optionally includes one or more features of any one or more of the previous aspects, wherein the environment comprises an assisted living facility.
7. A system, which optionally includes one or more features of any one or more of the previous aspects, wherein the environment comprises a campus.
8. A system, which optionally includes one or more features of any one or more of the previous aspects, wherein the environment comprises a neighbourhood.
9. A system, which optionally includes one or more features of any one or more of the previous aspects, wherein the environment comprises a town.
10. A system, which optionally includes one or more features of any one or more of the previous aspects, wherein the environment comprises a city.
11. A system, which optionally includes one or more features of any one or more of the previous aspects, wherein the environment comprises a province.
12. A system, which optionally includes one or more features of any one or more of the previous aspects, wherein the environment comprises a country.
13. A system, which optionally includes one or more features of any one or more of the previous aspects, wherein the environment comprises a region.
14. A system, which optionally includes one or more features of any one or more of the previous aspects, wherein the environment comprises a building and the zones comprise areas within the building.
15. A system, which optionally includes one or more features of any one or more of the previous aspects, wherein the environment comprises a building and the zones comprise rooms within the building.
16. A system, which optionally includes one or more features of any one or more of the previous aspects, wherein the environment comprises a building and the zones comprise floors within the building.
17. A system, which optionally includes one or more features of any one or more of the previous aspects, wherein the building comprises a hospital.
18. A system, which optionally includes one or more features of any one or more of the previous aspects, wherein the building comprises an assisted living facility.
19. A system, which optionally includes one or more features of any one or more of the previous aspects, wherein the zones comprise areas within the campus.
20. A system, which optionally includes one or more features of any one or more of the previous aspects, wherein the zones comprise areas within the neighbourhood.
21. A system, which optionally includes one or more features of any one or more of the previous aspects, wherein the zones comprise areas within the town.
22. A system, which optionally includes one or more features of any one or more of the previous aspects, wherein the zones comprise areas within the city.
23. A system, which optionally includes one or more features of any one or more of the previous aspects, wherein the zones comprise areas within the province.
24. A system, which optionally includes one or more features of any one or more of the previous aspects, wherein the zones comprise areas within the country.
25. A system, which optionally includes one or more features of any one or more of the previous aspects, wherein the zones comprise areas within the region.
26. A system, which optionally includes one or more features of any one or more of the previous aspects, wherein the zones comprise buildings.
27. A system, which optionally includes one or more features of any one or more of the previous aspects, wherein the system is configured to record infections detected by the system over time.
28. A system, which optionally includes one or more features of any one or more of the previous aspects, wherein the system is configured to determine a path that the infections have spread through the zones of the environment over time based on the locations of the fluid dispensers where the infections were detected over time.
29. A system, which optionally includes one or more features of any one or more of the previous aspects, wherein the system is configured to predict where the infections will spread through the zones of the environment over time.
30. A system, which optionally includes one or more features of any one or more of the previous aspects, wherein the prediction is based at least in part on the path that the system has determined that the infections have spread through the zones of the environment over time.
31. A system, which optionally includes one or more features of any one or more of the previous aspects, wherein the prediction is based at least in part on a previous path that the system has determined that a previous infection has spread through the zones of the environment over time.
32. A system, which optionally includes one or more features of any one or more of the previous aspects, wherein the prediction is based at least in part on historical data of how past infections have spread through the environment over time.
33. A system, which optionally includes one or more features of any one or more of the previous aspects, wherein the prediction is based at least in part on known patterns of movement of people within the environment.
34. A system, which optionally includes one or more features of any one or more of the previous aspects, wherein the prediction is based at least in part on predicted patterns of movement of people within the environment.
35. A system, which optionally includes one or more features of any one or more of the previous aspects, wherein, when the system detects an infection, the system is configured to provide a notification at the fluid dispenser where the infection was detected.
36. A system, which optionally includes one or more features of any one or more of the previous aspects, wherein the system is configured to determine an identity of at least some users of the fluid dispensers.
37. A system, which optionally includes one or more features of any one or more of the previous aspects, wherein, when the system detects an infection, the system is configured to determine the identity of the user of the fluid dispenser at the time and location where the infection was detected.
38. A system, which optionally includes one or more features of any one or more of the previous aspects, wherein the system is configured to identify the user based at least in part on facial recognition.
39. A system, which optionally includes one or more features of any one or more of the previous aspects, wherein the system is configured to identify the user based at least in part on communications between the fluid dispenser and a device carried by the user.
40. A system, which optionally includes one or more features of any one or more of the previous aspects, wherein the device comprises at least one of: an identification; a smartphone; and a smart watch.
41. A system, which optionally includes one or more features of any one or more of the previous aspects, wherein the system is configured to identify the user based at least in part on an analysis of a skin microbiome of the user.
42. A system, which optionally includes one or more features of any one or more of the previous aspects, wherein the system is configured to notify a facility administrator of the identity of the user.
43. A system, which optionally includes one or more features of any one or more of the previous aspects, wherein the system is configured to track movements of the user over time.
44. A system, which optionally includes one or more features of any one or more of the previous aspects, wherein the system is configured to notify a public health authority of the identity of the user.
45. A system, which optionally includes one or more features of any one or more of the previous aspects, wherein the system is configured to notify the user of public health recommendations.
46. A system, which optionally includes one or more features of any one or more of the previous aspects, wherein, when the system detects an infection, the system is configured to provide instructions to the user who was using the fluid dispenser at the time and location where the infection was detected to perform an action.
47. A system, which optionally includes one or more features of any one or more of the previous aspects, wherein the action comprises exiting a facility.
48. A system, which optionally includes one or more features of any one or more of the previous aspects, wherein the action comprises sanitizing the user's hands.
49. A system, which optionally includes one or more features of any one or more of the previous aspects, wherein the action comprises wearing a mask.
50. A system, which optionally includes one or more features of any one or more of the previous aspects, wherein the action comprises isolating.
51. A system, which optionally includes one or more features of any one or more of the previous aspects, wherein the action comprises seeking medical attention.
52. A system, which optionally includes one or more features of any one or more of the previous aspects, wherein the action comprises performing a medical test.
53. A system, which optionally includes one or more features of any one or more of the previous aspects, wherein the instructions are communicated to a communication device carried by the user.
54. A system, which optionally includes one or more features of any one or more of the previous aspects, wherein the communication device comprises at least one of: a smart phone and a smart watch.
55. A system, which optionally includes one or more features of any one or more of the previous aspects, wherein, when the system detects an infection, the system is configured to provide a notification in the zone where the infection was detected.
56. A system, which optionally includes one or more features of any one or more of the previous aspects, wherein the notification comprises a visual and/or audible signal that is provided by at least one of the fluid dispensers in the zone where the infection was detected.
57. A system, which optionally includes one or more features of any one or more of the previous aspects, wherein, when the system detects an infection, the system is configured to provide a notification in one or more of the zones where the system predicts that the infection will spread.
58. A system, which optionally includes one or more features of any one or more of the previous aspects, wherein the notification comprises a visual and/or audible signal that is provided by at least one of the fluid dispensers in the one or more zones where the system predicts that the infection will spread.
59. A system, which optionally includes one or more features of any one or more of the previous aspects, wherein, when the system detects an infection, the system is configured to provide a notification to an administrator of a facility where the infection was detected.
60. A system, which optionally includes one or more features of any one or more of the previous aspects, wherein, when the system detects an infection, the system is configured to provide a notification to a public health authority.
61. A system, which optionally includes one or more features of any one or more of the previous aspects, wherein, when the system detects an infection, the system is configured to modify an operation of the fluid dispenser where the infection was detected.
62. A system, which optionally includes one or more features of any one or more of the previous aspects, wherein, when the system detects an infection, the system is configured to modify an operation of at least some of the fluid dispensers.
63. A system, which optionally includes one or more features of any one or more of the previous aspects, wherein, when the system detects an infection, the system is configured to modify an operation of at least some of the fluid dispensers that are located in the zone where the infection was detected.
64. A system, which optionally includes one or more features of any one or more of the previous aspects, wherein, when the system detects an infection, the system is configured to modify an operation of at least some of the fluid dispensers that are located in the zone or zones where the system predicts that the infection will spread.
65. A system, which optionally includes one or more features of any one or more of the previous aspects, wherein the modification comprises providing a notification that an infection has been detected.
66. A system, which optionally includes one or more features of any one or more of the previous aspects, wherein the modification comprises providing a notification that the spread of an infection to the zone or zones is predicted.
67. A system, which optionally includes one or more features of any one or more of the previous aspects, wherein the modification comprises providing a signal encouraging hand cleaning.
68. A system, which optionally includes one or more features of any one or more of the previous aspects, wherein the modification comprises a change in a formulation of the hand cleaning fluid.
69. A system, which optionally includes one or more features of any one or more of the previous aspects, wherein the modification comprises a change in a volume of the hand cleaning fluid that is dispensed.
70. A system, which optionally includes one or more features of any one or more of the previous aspects, wherein at least some of the plurality of fluid dispensers have a fluid analysis mechanism for analyzing at least some of the hand cleaning fluid after the hand cleaning fluid has contacted the user's hand; and wherein the modification comprises a change in an operation of the fluid analysis mechanism.
71. A system, which optionally includes one or more features of any one or more of the previous aspects, wherein, before the system detects an infection, at least some of the fluid analysis mechanisms of the fluid dispensers are inactivated; and wherein the modification comprises activating at least some of the fluid analysis mechanisms.
72. A system, which optionally includes one or more features of any one or more of the previous aspects, wherein at least some of the fluid dispensers comprise a fluid analysis mechanism for analyzing at least some of the hand cleaning fluid after the hand cleaning fluid has contacted the user's hand, the fluid analysis mechanism comprising: a fluid collector for collecting at least some of the hand cleaning fluid after the hand cleaning fluid has contacted the user's hand; an analysis chamber that is fluidly connected to the fluid collector for receiving at least some of the hand cleaning fluid collected by the fluid collector; and a camera that captures at least one image of the hand cleaning fluid received by the analysis chamber.
73. A system, which optionally includes one or more features of any one or more of the previous aspects, wherein at least some of the fluid analysis mechanisms comprise: a fluid collector for collecting at least some of the hand cleaning fluid after the hand cleaning fluid has contacted the user's hand; an analysis chamber that is fluidly connected to the fluid collector for receiving at least some of the hand cleaning fluid collected by the fluid collector; and a camera that captures at least one image of the hand cleaning fluid received by the analysis chamber.
74. A system, which optionally includes one or more features of any one or more of the previous aspects, wherein the system is configured to detect particles in the at least one image.
75. A system, which optionally includes one or more features of any one or more of the previous aspects, wherein the system is configured to identify the particles.
76. A system, which optionally includes one or more features of any one or more of the previous aspects, wherein the system is configured to categorize the particles into predetermined classes.
77. A system, which optionally includes one or more features of any one or more of the previous aspects, wherein the predetermined classes comprise at least one recognized class and at least one unrecognized class; wherein the system is configured to categorize the particles that meet predetermined criteria into the at least one recognized class; and wherein the system is configured to categorize the particles that fail to meet the predetermined criteria into the at least one unrecognized class.
78. A system, which optionally includes one or more features of any one or more of the previous aspects, wherein the system comprises a machine learning algorithm that is configured to select the predetermined criteria based on training data.
79. A system, which optionally includes one or more features of any one or more of the previous aspects, wherein the training data comprises images of the hand cleaning fluid captured by the camera during a training operation.
80. A system, which optionally includes one or more features of any one or more of the previous aspects, wherein the system is configured to determine a background rate of the particles that are categorized into each of the at least one recognized class and each of the at least one unrecognized class based at least in part on images of the hand cleaning fluid captured by the camera over time.
81. A system, which optionally includes one or more features of any one or more of the previous aspects, wherein the system is configured to detect the infection based at least in part on detection of an increase in a quantity of the particles that are categorized into at least one of the at least one recognized class and the at least one unrecognized class above the background rate.
82. A system, which optionally includes one or more features of any one or more of the previous aspects, wherein the system is configured to detect the infection based at least in part on detection of an increase in a quantity of the particles that are categorized into at least one of the at least one recognized class and the at least one unrecognized class by at least a threshold amount above the background rate.
83. A system, which optionally includes one or more features of any one or more of the previous aspects, wherein the predetermined criteria comprise a size of the particles.
84. A system, which optionally includes one or more features of any one or more of the previous aspects, wherein the predetermined criteria comprise a shape of the particles.
85. A system, which optionally includes one or more features of any one or more of the previous aspects, wherein the predetermined criteria comprise a movement of the particles.
86. A system, which optionally includes one or more features of any one or more of the previous aspects, wherein the camera is configured to capture a series of images of the hand cleaning fluid received by the analysis chamber.
87. A system, which optionally includes one or more features of any one or more of the previous aspects, wherein the system is configured to track movement of the particles in the analysis chamber over time in the series of images.
88. A system, which optionally includes one or more features of any one or more of the previous aspects, wherein the predetermined criteria comprise the movement of the particles over time.
89. A system, which optionally includes one or more features of any one or more of the previous aspects, wherein the fluid analysis mechanism comprises a stop mechanism that stops a flow of the hand cleaning fluid through the analysis chamber.
90. A system, which optionally includes one or more features of any one or more of the previous aspects, wherein at least some of the series of images are captured after the flow of the hand cleaning fluid through the analysis chamber has stopped.
91. A system, which optionally includes one or more features of any one or more of the previous aspects, wherein the system is configured to identify the particles as active or inactive based at least in part on the movement of the particles over time.
92. A system, which optionally includes one or more features of any one or more of the previous aspects, wherein the system is configured to identify the particles as inactive based at least in part on sedimentation of the particles.
93. A system, which optionally includes one or more features of any one or more of the previous aspects, wherein the system is configured to calculate a sanitation effectiveness value based at least in part on a comparison of a quantity of the particles that are identified as active and a quantity of the particles that are identified as inactive.
94. A system, which optionally includes one or more features of any one or more of the previous aspects, wherein the system is configured to record the sanitation effectiveness value.
95. A system, which optionally includes one or more features of any one or more of the previous aspects, wherein the system is configured to provide a notification of the sanitation effectiveness value.
96. A system, which optionally includes one or more features of any one or more of the previous aspects, wherein the system is configured to modify an operation of the fluid dispenser if the sanitation effectiveness value falls below a predetermined standard.
97. A system, which optionally includes one or more features of any one or more of the previous aspects, wherein the modification comprises changing a formulation of the hand cleaning fluid that is dispensed.
98. A system, which optionally includes one or more features of any one or more of the previous aspects, wherein the modification comprises changing a volume of the hand cleaning fluid that is dispensed.
99. A system, which optionally includes one or more features of any one or more of the previous aspects, wherein the camera captures the at least one image of the hand cleaning fluid from above the analysis chamber.
100. A system, which optionally includes one or more features of any one or more of the previous aspects, wherein the camera captures the at least one image of the hand cleaning fluid from a side of the analysis chamber.
101. A system, which optionally includes one or more features of any one or more of the previous aspects, wherein the camera captures the at least one image of the hand cleaning fluid from below the analysis chamber.
102. A system, which optionally includes one or more features of any one or more of the previous aspects, wherein the analysis chamber comprises an analysis channel.
103. A system, which optionally includes one or more features of any one or more of the previous aspects, wherein the analysis chamber comprises an analysis cone.
104. A system, which optionally includes one or more features of any one or more of the previous aspects, wherein the analysis cone has an apex, a base, and an internal cavity; wherein the internal cavity is in fluid communication with a fluid inlet located at the apex; and wherein the internal cavity is in fluid communication with a plurality of fluid outlets located around a periphery of the base.
105. A system, which optionally includes one or more features of any one or more of the previous aspects, wherein a height of the internal cavity decreases as the internal cavity extends radially outwardly from the apex.
106. A system, which optionally includes one or more features of any one or more of the previous aspects, wherein the internal cavity is defined between an upper surface and a lower surface; and wherein a distance between the upper surface and the lower surface decreases as the internal cavity extends radially outwardly from the apex.
107. A system, which optionally includes one or more features of any one or more of the previous aspects, wherein the predetermined criteria comprise a location where the particles become lodged between the upper surface and the lower surface.
108. A system, which optionally includes one or more features of any one or more of the previous aspects, wherein the predetermined criteria comprise a distance that the particles drift after the flow is stopped.
109. A system, which optionally includes one or more features of any one or more of the previous aspects, wherein the system is configured to periodically clean the analysis chamber by passing a cleaning fluid through the analysis chamber.
110. A system, which optionally includes one or more features of any one or more of the previous aspects, wherein the cleaning fluid comprises the hand cleaning fluid.
111. A system, which optionally includes one or more features of any one or more of the previous aspects, wherein the cleaning fluid is passed through the analysis chamber in a direction that is opposite to a flow direction of the hand cleaning fluid during the analysis.
112. A system, which optionally includes one or more features of any one or more of the previous aspects, wherein the fluid analysis mechanism comprises a pump that pumps the hand cleaning fluid through the analysis chamber.
113. A system, which optionally includes one or more features of any one or more of the previous aspects, wherein the flow of the hand cleaning fluid through the analysis chamber is stopped by deactivating the pump.
114. A system, which optionally includes one or more features of any one or more of the previous aspects, wherein the stop mechanism is configured to equalize a fluid pressure upstream of the analysis chamber and downstream of the analysis chamber, so as to stop the flow of the hand cleaning fluid through the analysis chamber.
115. A system, which optionally includes one or more features of any one or more of the previous aspects, wherein the stop mechanism comprises a valve.
116. A system, which optionally includes one or more features of any one or more of the previous aspects, wherein the valve is moveable to a stop position, in which the valve is open to atmospheric air.
117. A system, which optionally includes one or more features of any one or more of the previous aspects, wherein the stop mechanism comprises an upstream valve, a downstream valve, and an equalization conduit; wherein the upstream valve is located upstream of the analysis chamber; wherein the downstream valve is located downstream of the analysis chamber; wherein the upstream valve has a flow position, in which the analysis chamber is fluidly disconnected from the equalization conduit via the upstream valve; wherein the downstream valve has a flow position, in which the analysis chamber is fluidly disconnected from the equalization conduit via the downstream valve; wherein the upstream valve has a stop position, in which the analysis chamber is in fluid communication with the equalization conduit via the upstream valve; and wherein the downstream valve has a stop position, in which the analysis chamber is in fluid communication with the equalization conduit via the downstream valve.
118. A system, which optionally includes one or more features of any one or more of the previous aspects, the fluid dispensers are configured to independently detect an infection.
119. A system, which optionally includes one or more features of any one or more of the previous aspects, wherein, when one of the fluid dispensers detects an infection, the fluid dispenser is configured to send a signal to at least another one of the fluid dispensers; and wherein the signal indicates that the fluid dispenser has detected the infection.
120. A system, which optionally includes one or more features of any one or more of the previous aspects, wherein the system comprises at least one server that communicates with at least some of the fluid dispensers.
121. A system, which optionally includes one or more features of any one or more of the previous aspects, wherein the at least one server receives external data from at least one external source.
122. A system, which optionally includes one or more features of any one or more of the previous aspects, wherein the external data comprises public health information.
123. A system, which optionally includes one or more features of any one or more of the previous aspects, wherein the external data comprises traffic data.
124. A system, which optionally includes one or more features of any one or more of the previous aspects, wherein the external data comprises transit data.
125. A system, which optionally includes one or more features of any one or more of the previous aspects, wherein the at least one server determines the path that the infections have spread through the zones of the environment over time.
126. A system, which optionally includes one or more features of any one or more of the previous aspects, wherein the at least one server predicts where the infections will spread through the zones of the environment over time.
127. A system, which optionally includes one or more features of any one or more of the previous aspects, wherein the prediction is based at least in part on the external data.
128. A system, which optionally includes one or more features of any one or more of the previous aspects, wherein the at least one server determines whether there is an elevated prevalence of infections in the environment based at least in part on data received from at least some of the fluid dispensers.
129. A system, which optionally includes one or more features of any one or more of the previous aspects, wherein the at least one server determines whether there is an elevated prevalence of infections in each of the zones of the environment based at least in part on data received from at least some of the fluid dispensers in each of the zones.
130. A system, which optionally includes one or more features of any one or more of the previous aspects, wherein the at least one server has a server-side machine learning algorithm that is configured to select a method of determining the path that the infections have spread through the zones of the environment over time based at least in part on an evaluation of past performance of the system.
131. A system, which optionally includes one or more features of any one or more of the previous aspects, wherein the at least one server has a server-side machine learning algorithm that is configured to select a method of predicting where the infections will spread through the zones of the environment over time based at least in part on an evaluation of past performance of the system.
132. A system, which optionally includes one or more features of any one or more of the previous aspects, wherein the at least one server has a server-side machine learning algorithm that is configured to select a method of determining whether there is an elevated prevalence of infections in the environment based at least in part on an evaluation of past performance of the system.
133. A system, which optionally includes one or more features of any one or more of the previous aspects, wherein the at least one server has a server-side machine learning algorithm that is configured to select a method of determining whether there is an elevated prevalence of infections in each of the zones of the environment based at least in part on an evaluation of past performance of the system.
134. A system, which optionally includes one or more features of any one or more of the previous aspects, wherein the system is configured to determine a travel history of the user, and to determine the path that the infection has spread through the zones of the environment over time based at least in part on the travel history.
135. A system, which optionally includes one or more features of any one or more of the previous aspects, wherein the system is configured to determine a travel history of the user, and to predict where the infections will spread through the zones of the environment over time based at least in part on the travel history.
136. A system, which optionally includes one or more features of any one or more of the previous aspects, wherein the system is configured to collect physiological data about at least some users of the fluid dispensers.
137. A system, which optionally includes one or more features of any one or more of the previous aspects, wherein the physiological data comprises body temperature data.
138. A system, which optionally includes one or more features of any one or more of the previous aspects, wherein the physiological data comprises heart rate data.
139. A system, which optionally includes one or more features of any one or more of the previous aspects, wherein the physiological data comprises blood oxygenation data.
140. A system, which optionally includes one or more features of any one or more of the previous aspects, wherein the physiological data is collected by a smart device.
141. A system, which optionally includes one or more features of any one or more of the previous aspects, wherein the smart device comprises a smart watch.
142. A system, which optionally includes one or more features of any one or more of the previous aspects, wherein the system detects the infection based at least in part on the physiological data.
143. A system, which optionally includes one or more features of any one or more of the previous aspects, wherein the system is configured to analyze the hand cleaning fluid using at least one of: electrical, acoustic, optical, magnetic, spectroscopic, electromagnetic, mechanical, thermal, and chemical methods.
144. A fluid dispenser, which optionally includes one or more features of any one or more of the previous aspects, comprising: a fluid reservoir containing a supply of hand cleaning fluid; a dispensing mechanism for dispensing the fluid onto a user's hand; and a fluid analysis mechanism for analyzing at least some of the hand cleaning fluid after the hand cleaning fluid has contacted the user's hand; wherein the fluid dispenser is configured to detect an infection based at least in part on the analysis of the hand cleaning fluid.
145. A fluid dispenser, which optionally includes one or more features of any one or more of the previous aspects, wherein, when the fluid dispenser detects an infection, the fluid dispenser is configured to provide a notification.
146. A fluid dispenser, which optionally includes one or more features of any one or more of the previous aspects, wherein the fluid dispenser is configured to determine an identity of at least some users of the fluid dispenser.
147. A fluid dispenser, which optionally includes one or more features of any one or more of the previous aspects, wherein, when the fluid dispenser detects an infection, the fluid dispenser is configured to determine the identity of the user who was using the fluid dispenser when the infection was detected.
148. A fluid dispenser, which optionally includes one or more features of any one or more of the previous aspects, wherein the fluid dispenser is configured to identify the user based at least in part on facial recognition.
149. A fluid dispenser, which optionally includes one or more features of any one or more of the previous aspects, wherein the fluid dispenser is configured to identify the user based at least in part on communications between the fluid dispenser and a device carried by the user.
150. A fluid dispenser, which optionally includes one or more features of any one or more of the previous aspects, wherein the device comprises at least one of: an identification; a smartphone; and a smart watch.
151. A fluid dispenser, which optionally includes one or more features of any one or more of the previous aspects, wherein the fluid dispenser is configured to identify the user based at least in part on an analysis of a skin microbiome of the user.
152. A fluid dispenser, which optionally includes one or more features of any one or more of the previous aspects, wherein the fluid dispenser is configured to notify a facility administrator of the identity of the user.
153. A fluid dispenser, which optionally includes one or more features of any one or more of the previous aspects, wherein the fluid dispenser is configured to notify a public health authority of the identity of the user.
154. A fluid dispenser, which optionally includes one or more features of any one or more of the previous aspects, wherein the fluid dispenser is configured to notify the user of public health recommendations.
155. A fluid dispenser, which optionally includes one or more features of any one or more of the previous aspects, wherein, when the fluid dispenser detects an infection, the fluid dispenser is configured to provide instructions to the user who was using the fluid dispenser when the infection was detected to perform an action.
156. A fluid dispenser, which optionally includes one or more features of any one or more of the previous aspects, wherein the action comprises exiting a facility.
157. A fluid dispenser, which optionally includes one or more features of any one or more of the previous aspects, wherein the action comprises sanitizing the user's hands.
158. A fluid dispenser, which optionally includes one or more features of any one or more of the previous aspects, wherein the action comprises wearing a mask.
159. A fluid dispenser, which optionally includes one or more features of any one or more of the previous aspects, wherein the action comprises isolating.
160. A fluid dispenser, which optionally includes one or more features of any one or more of the previous aspects, wherein the action comprises seeking medical attention.
161. A fluid dispenser, which optionally includes one or more features of any one or more of the previous aspects, wherein the action comprises performing a medical test.
162. A fluid dispenser, which optionally includes one or more features of any one or more of the previous aspects, wherein the instructions are communicated to a communication device carried by the user.
163. A fluid dispenser, which optionally includes one or more features of any one or more of the previous aspects, wherein the communication device comprises a smart phone.
164. A fluid dispenser, which optionally includes one or more features of any one or more of the previous aspects, wherein the notification comprises a visual and/or audible signal that is provided by the fluid dispenser.
165. A fluid dispenser, which optionally includes one or more features of any one or more of the previous aspects, wherein, when the fluid dispenser detects an infection, the fluid dispenser is configured to provide a notification to an administrator of a facility where the fluid dispenser is located.
166. A fluid dispenser, which optionally includes one or more features of any one or more of the previous aspects, wherein, when the fluid dispenser detects an infection, the fluid dispenser is configured to provide a notification to a public health authority.
167. A fluid dispenser, which optionally includes one or more features of any one or more of the previous aspects, wherein the communication device comprises a smart watch.
168. A fluid dispenser, which optionally includes one or more features of any one or more of the previous aspects, wherein, when the fluid dispenser detects an infection, the fluid dispenser is configured to modify an operation of the fluid dispenser.
169. A fluid dispenser, which optionally includes one or more features of any one or more of the previous aspects, wherein the modification comprises providing a notification that an infection has been detected.
170. A fluid dispenser, which optionally includes one or more features of any one or more of the previous aspects, wherein the modification comprises providing a signal encouraging hand cleaning.
171. A fluid dispenser, which optionally includes one or more features of any one or more of the previous aspects, wherein the modification comprises a change in a formulation of the hand cleaning fluid.
172. A fluid dispenser, which optionally includes one or more features of any one or more of the previous aspects, wherein the modification comprises a change in a volume of the hand cleaning fluid that is dispensed.
173. A fluid dispenser, which optionally includes one or more features of any one or more of the previous aspects, wherein the modification comprises a change in an operation of the fluid analysis mechanism.
174. A fluid dispenser, which optionally includes one or more features of any one or more of the previous aspects, wherein the fluid analysis mechanism comprises: a fluid collector for collecting at least some of the hand cleaning fluid after the hand cleaning fluid has contacted the user's hand; an analysis chamber that is fluidly connected to the fluid collector for receiving at least some of the hand cleaning fluid collected by the fluid collector; and a camera that captures at least one image of the hand cleaning fluid received by the analysis chamber.
175. A fluid dispenser, which optionally includes one or more features of any one or more of the previous aspects, wherein the fluid dispenser is configured to detect particles in the at least one image.
176. A fluid dispenser, which optionally includes one or more features of any one or more of the previous aspects, wherein the fluid dispenser is configured to identify the particles.
177. A fluid dispenser, which optionally includes one or more features of any one or more of the previous aspects, wherein the fluid dispenser is configured to categorize the particles into predetermined classes.
178. A fluid dispenser, which optionally includes one or more features of any one or more of the previous aspects, wherein the predetermined classes comprise at least one recognized class and at least one unrecognized class; wherein the fluid dispenser is configured to categorize the particles that meet predetermined criteria into the at least one recognized class; and wherein the fluid dispenser is configured to categorize the particles that fail to meet the predetermined criteria into the at least one unrecognized class.
179. A fluid dispenser, which optionally includes one or more features of any one or more of the previous aspects, wherein the fluid dispenser comprises a machine learning algorithm that is configured to select the predetermined criteria based on training data.
180. A fluid dispenser, which optionally includes one or more features of any one or more of the previous aspects, wherein the training data comprises images of the hand cleaning fluid captured by the camera during a training operation.
181. A fluid dispenser, which optionally includes one or more features of any one or more of the previous aspects, wherein the fluid dispenser is configured to determine a background rate of the particles that are categorized into each of the at least one recognized class and each of the at least one unrecognized class based at least in part on images of the hand cleaning fluid captured by the camera over time.
182. A fluid dispenser, which optionally includes one or more features of any one or more of the previous aspects, wherein the fluid dispenser is configured to detect the infection based at least in part on detection of an increase in a quantity of the particles that are categorized into at least one of the at least one recognized class and the at least one unrecognized class above the background rate.
183. A fluid dispenser, which optionally includes one or more features of any one or more of the previous aspects, wherein the fluid dispenser is configured to detect the infection based at least in part on detection of an increase in a quantity of the particles that are categorized into at least one of the at least one recognized class and the at least one unrecognized class by at least a threshold amount above the background rate.
184. A fluid dispenser, which optionally includes one or more features of any one or more of the previous aspects, wherein the predetermined criteria comprise a size of the particles.
185. A fluid dispenser, which optionally includes one or more features of any one or more of the previous aspects, wherein the predetermined criteria comprise a shape of the particles.
186. A fluid dispenser, which optionally includes one or more features of any one or more of the previous aspects, wherein the predetermined criteria comprise a movement of the particles.
187. A fluid dispenser, which optionally includes one or more features of any one or more of the previous aspects, wherein the camera is configured to capture a series of images of the hand cleaning fluid received by the analysis chamber.
188. A fluid dispenser, which optionally includes one or more features of any one or more of the previous aspects, wherein the fluid dispenser is configured to track movement of the particles in the analysis chamber over time in the series of images.
189. A fluid dispenser, which optionally includes one or more features of any one or more of the previous aspects, wherein the predetermined criteria comprise the movement of the particles over time.
190. A fluid dispenser, which optionally includes one or more features of any one or more of the previous aspects, wherein the fluid analysis mechanism comprises a stop mechanism that stops a flow of the hand cleaning fluid through the analysis chamber.
191. A fluid dispenser, which optionally includes one or more features of any one or more of the previous aspects, wherein at least some of the series of images are captured after the flow of the hand cleaning fluid through the analysis chamber has stopped.
192. A fluid dispenser, which optionally includes one or more features of any one or more of the previous aspects, wherein the fluid dispenser is configured to identify the particles as active or inactive based at least in part on the movement of the particles over time.
193. A fluid dispenser, which optionally includes one or more features of any one or more of the previous aspects, wherein the fluid dispenser is configured to identify the particles as inactive based at least in part on sedimentation of the particles.
194. A fluid dispenser, which optionally includes one or more features of any one or more of the previous aspects, wherein the fluid dispenser is configured to calculate a sanitation effectiveness value based at least in part on a comparison of a quantity of the particles that are identified as active and a quantity of the particles that are identified as inactive.
195. A fluid dispenser, which optionally includes one or more features of any one or more of the previous aspects, wherein the fluid dispenser is configured to record the sanitation effectiveness value.
196. A fluid dispenser, which optionally includes one or more features of any one or more of the previous aspects, wherein the fluid dispenser is configured to provide a notification of the sanitation effectiveness value.
197. A fluid dispenser, which optionally includes one or more features of any one or more of the previous aspects, wherein the fluid dispenser is configured to modify an operation of the fluid dispenser if the sanitation effectiveness value falls below a predetermined standard.
198. A fluid dispenser, which optionally includes one or more features of any one or more of the previous aspects, wherein the modification comprises changing a formulation of the hand cleaning fluid that is dispensed.
199. A fluid dispenser, which optionally includes one or more features of any one or more of the previous aspects, wherein the modification comprises changing a volume of the hand cleaning fluid that is dispensed.
200. A fluid dispenser, which optionally includes one or more features of any one or more of the previous aspects, wherein the camera captures the at least one image of the hand cleaning fluid from above the analysis chamber.
201. A fluid dispenser, which optionally includes one or more features of any one or more of the previous aspects, wherein the camera captures the at least one image of the hand cleaning fluid from a side of the analysis chamber.
202. A fluid dispenser, which optionally includes one or more features of any one or more of the previous aspects, wherein the camera captures the at least one image of the hand cleaning fluid from below the analysis chamber.
203. A fluid dispenser, which optionally includes one or more features of any one or more of the previous aspects, wherein the analysis chamber comprises an analysis channel.
204. A fluid dispenser, which optionally includes one or more features of any one or more of the previous aspects, wherein the analysis chamber comprises an analysis cone.
205. A fluid dispenser, which optionally includes one or more features of any one or more of the previous aspects, wherein the analysis cone has an apex, a base, and an internal cavity; wherein the internal cavity is in fluid communication with a fluid inlet located at the apex; and wherein the internal cavity is in fluid communication with a plurality of fluid outlets located around a periphery of the base.
206. A fluid dispenser, which optionally includes one or more features of any one or more of the previous aspects, wherein a height of the internal cavity decreases as the internal cavity extends radially outwardly from the apex.
207. A fluid dispenser, which optionally includes one or more features of any one or more of the previous aspects, wherein the internal cavity is defined between an upper surface and a lower surface; and wherein a distance between the upper surface and the lower surface decreases as the internal cavity extends radially outwardly from the apex.
208. A fluid dispenser, which optionally includes one or more features of any one or more of the previous aspects, wherein the predetermined criteria comprise a location where the particles become lodged between the upper surface and the lower surface.
209. A fluid dispenser, which optionally includes one or more features of any one or more of the previous aspects, wherein the predetermined criteria comprise a distance that the particles drift after the flow is stopped.
210. A fluid dispenser, which optionally includes one or more features of any one or more of the previous aspects, wherein the fluid dispenser is configured to periodically clean the analysis chamber by passing a cleaning fluid through the analysis chamber.
211. A fluid dispenser, which optionally includes one or more features of any one or more of the previous aspects, wherein the cleaning fluid comprises the hand cleaning fluid.
212. A fluid dispenser, which optionally includes one or more features of any one or more of the previous aspects, wherein the cleaning fluid is passed through the analysis chamber in a direction that is opposite to a flow direction of the hand cleaning fluid during the analysis.
213. A fluid dispenser, which optionally includes one or more features of any one or more of the previous aspects, wherein the fluid analysis mechanism comprises a pump that pumps the hand cleaning fluid through the analysis chamber.
214. A fluid dispenser, which optionally includes one or more features of any one or more of the previous aspects, wherein the flow of the hand cleaning fluid through the analysis chamber is stopped by deactivating the pump.
215. A fluid dispenser, which optionally includes one or more features of any one or more of the previous aspects, wherein the stop mechanism is configured to equalize a fluid pressure upstream of the analysis chamber and downstream of the analysis chamber, so as to stop the flow of the hand cleaning fluid through the analysis chamber.
216. A fluid dispenser, which optionally includes one or more features of any one or more of the previous aspects, wherein the stop mechanism comprises a valve.
217. A fluid dispenser, which optionally includes one or more features of any one or more of the previous aspects, wherein the valve is moveable to a stop position, in which the valve is open to atmospheric air.
218. A fluid dispenser, which optionally includes one or more features of any one or more of the previous aspects, wherein the stop mechanism comprises an upstream valve, a downstream valve, and an equalization conduit; wherein the upstream valve is located upstream of the analysis chamber; wherein the downstream valve is located downstream of the analysis chamber; wherein the upstream valve has a flow position, in which the analysis chamber is fluidly disconnected from the equalization conduit via the upstream valve; wherein the downstream valve has a flow position, in which the analysis chamber is fluidly disconnected from the equalization conduit via the downstream valve; wherein the upstream valve has a stop position, in which the analysis chamber is in fluid communication with the equalization conduit via the upstream valve; and wherein the downstream valve has a stop position, in which the analysis chamber is in fluid communication with the equalization conduit via the downstream valve.
219. A fluid dispenser, which optionally includes one or more features of any one or more of the previous aspects, wherein the fluid dispenser is configured to independently detect an infection.
220. A fluid dispenser, which optionally includes one or more features of any one or more of the previous aspects, wherein, when the fluid dispenser detects an infection, the fluid dispenser is configured to send a signal to at least one other fluid dispenser; and wherein the signal indicates that the fluid dispenser has detected the infection.
221. A fluid dispenser, which optionally includes one or more features of any one or more of the previous aspects, wherein, when the fluid dispenser detects an infection, the fluid dispenser is configured to send a signal to at least one server; and wherein the signal indicates that the fluid dispenser has detected the infection.
220. A fluid dispenser, which optionally includes one or more features of any one or more of the previous aspects, wherein the fluid dispenser is configured to collect physiological data about at least some users of the fluid dispenser.
221. A fluid dispenser, which optionally includes one or more features of any one or more of the previous aspects, wherein the physiological data comprises body temperature data.
222. A fluid dispenser, which optionally includes one or more features of any one or more of the previous aspects, wherein the physiological data comprises heart rate data.
223. A fluid dispenser, which optionally includes one or more features of any one or more of the previous aspects, wherein the physiological data comprises blood oxygenation data.
224. A fluid dispenser, which optionally includes one or more features of any one or more of the previous aspects, wherein fluid dispenser is configured to receive the physiological data from a smart device.
225. A fluid dispenser, which optionally includes one or more features of any one or more of the previous aspects, wherein the smart device comprises a smart watch.
226. A fluid dispenser, which optionally includes one or more features of any one or more of the previous aspects, wherein the fluid dispenser detects the infection based at least in part on the physiological data.
227. A fluid dispenser, which optionally includes one or more features of any one or more of the previous aspects, wherein the fluid dispenser is configured to analyze the hand cleaning fluid using at least one of: electrical, acoustic, optical, magnetic, spectroscopic, electromagnetic, mechanical, thermal, and chemical methods.
228. A system, which optionally includes one or more features of any one or more of the previous aspects, wherein at least one of the fluid dispensers comprises the fluid dispenser in accordance with one or more of the previous aspects.
229. A method, which optionally includes one or more features of any one or more of the previous aspects, comprising: providing a fluid dispenser for dispensing hand cleaning fluid; analyzing at least some of the hand cleaning fluid after the hand cleaning fluid has contacted a user's hand; and detecting an infection based at least in part on the analysis of the hand cleaning fluid.
230. A method, which optionally includes one or more features of any one or more of the previous aspects, wherein the fluid dispenser comprises the fluid dispenser in accordance with one or more of the previous aspects.
231. A method, which optionally includes one or more features of any one or more of the previous aspects, wherein the method is performed by the system in accordance with one or more of the previous aspects.
232. A fluid dispenser, a method, or a system, which optionally includes one or more features of any one or more of the previous aspects, wherein the particles are categorized based at least in part on movement of the particles in three dimensions.
233. A fluid dispenser, a method, or a system, which optionally includes one or more features of any one or more of the previous aspects, wherein the movement of the particles in three dimensions includes three dimensional velocity and three dimensional acceleration.
234. A fluid dispenser, a method, or a system, which optionally includes one or more features of any one or more of the previous aspects, wherein at least some of the fluid dispensers comprise a fluid analysis mechanism for analyzing at least some of the hand cleaning fluid after the hand cleaning fluid has contacted the user's hand, the fluid analysis mechanism comprising: at least one detection device that captures data about the particles present in the hand cleaning fluid; wherein the fluid dispenser is configured to categorize the particles into predetermined classes based at least in part on the data.
235. A fluid dispenser, a method, or a system according to any one or more of the previous aspects, wherein the term "camera" is replaced by "at least one detection device".
236. A fluid dispenser, a method, or a system according to any one or more of the previous aspects, wherein the term "image" is replaced by "data" or "data sets".
237. A fluid dispenser, a method, or a system, which optionally includes one or more features of any one or more of the previous aspects, wherein the predetermined criteria comprise a cluster characteristic of the particles.
238. A fluid dispenser, a method, or a system, which optionally includes one or more features of any one or more of the previous aspects, wherein the particles are identified as inactive based at least in part on a deceleration or stopping of the particles over time.
239. A fluid dispenser, a method, or a system, which optionally includes one or more features of any one or more of the previous aspects, wherein the particles are identified as inactive based at least in part on a deceleration or stopping of the particles over time caused by at least one of: sedimentation under gravity and hydrodynamic resistance.
240. A system, which optionally includes one or more features of any one or more of the previous aspects, comprising: a plurality of fluid dispensers for dispensing hand cleaning fluid; wherein at least some of the fluid dispensers are configured to analyze at least some of the hand cleaning fluid after the hand cleaning fluid has contacted a user's hand; and wherein the system is configured to detect an infection based at least in part on the analysis of the hand cleaning fluid.
241. A system, which optionally includes one or more features of any one or more of the previous aspects, wherein the plurality of fluid dispensers are located at a plurality of different locations within an environment; wherein the environment has a plurality of different zones; wherein at least one of the fluid dispensers is located in each of the zones; and wherein, when the system detects an infection, the system is configured to identify which of the zones the infection is located in based on the location of the fluid dispenser where the infection was detected.
242. A system, which optionally includes one or more features of any one or more of the previous aspects, wherein the system is configured to record infections detected by the system over time; and wherein the system is configured to determine a path that the infections have spread through the zones of the environment over time based on the locations of the fluid dispensers where the infections were detected over time.
243. A system, which optionally includes one or more features of any one or more of the previous aspects, wherein the system is configured to predict where the infections will spread through the zones of the environment over time.
244. A system, which optionally includes one or more features of any one or more of the previous aspects, wherein the prediction is based at least in part on one or more of: the path that the system has determined that the infections have spread through the zones of the environment over time; a previous path that the system has determined that a previous infection has spread through the zones of the environment over time; historical data of how past infections have spread through the environment over time; known patterns of movement of people within the environment; and predicted patterns of movement of people within the environment.
245. A system, which optionally includes one or more features of any one or more of the previous aspects, wherein, when the system detects an infection, the system is configured to determine an identity of the user of the fluid dispenser where the infection was detected; and wherein the system is configured to at least one of: identify the user based at least in part on facial recognition; identify the user based at least in part on communications between the fluid dispenser and a device carried by the user; identify the user based at least in part on an analysis of a skin microbiome of the user; notify a facility administrator of the identity of the user; track movements of the user over time; and notify a public health authority of the identity of the user.
246. A system, which optionally includes one or more features of any one or more of the previous aspects, wherein, when the system detects an infection, the system is configured to provide instructions to the user of the fluid dispenser where the infection was detected to perform an action; wherein the instructions are communicated to a communication device carried by the user; and wherein the action comprises at least one of: exiting a facility; sanitizing the user's hands; wearing a mask; isolating; seeking medical attention; and performing a medical test.
247. A system, which optionally includes one or more features of any one or more of the previous aspects, wherein, when the system detects an infection, the system is configured to provide a notification in the zone where the infection was detected; and wherein the notification comprises a visual and/or audible signal that is provided by at least one of the fluid dispensers in the zone where the infection was detected.
248. A system, which optionally includes one or more features of any one or more of the previous aspects, wherein, when the system detects an infection, the system is configured to provide a notification in one or more of the zones where the system predicts that the infection will spread; and wherein the notification comprises a visual and/or audible signal that is provided by at least one of the fluid dispensers in the one or more zones where the system predicts that the infection will spread.
249. A system, which optionally includes one or more features of any one or more of the previous aspects, wherein, when the system detects an infection, the system is configured to modify an operation of at least one of the fluid dispensers; and wherein the modification comprises at least one of: providing a signal encouraging hand cleaning; changing a formulation of the hand cleaning fluid; changing a volume of the hand cleaning fluid that is dispensed; and changing an operation of a fluid analysis mechanism.
250. A system, which optionally includes one or more features of any one or more of the previous aspects, wherein at least some of the fluid dispensers have a fluid analysis mechanism; wherein, in at least one mode of operation of the system, a subset of the fluid analysis mechanisms are inactivated; and wherein, when the system detects an infection, the system is configured to activate at least some of the fluid analysis mechanisms that were previously inactivated.
251. A system, which optionally includes one or more features of any one or more of the previous aspects, wherein at least some of the fluid dispensers have a fluid analysis mechanism for analyzing at least some of the hand cleaning fluid after the hand cleaning fluid has contacted the user's hand, the fluid analysis mechanism comprising: at least one detection device that captures data about particles present in the hand cleaning fluid; wherein the system is configured to categorize the particles into predetermined classes based at least in part on the data; wherein the predetermined classes comprise at least one recognized class and at least one unrecognized class; wherein the system is configured to categorize the particles that meet predetermined criteria into the at least one recognized class; and wherein the system is configured to categorize the particles that fail to meet the predetermined criteria into the at least one unrecognized class.
252. A system, which optionally includes one or more features of any one or more of the previous aspects, wherein at least some of the fluid dispensers have a fluid analysis mechanism for analyzing at least some of the hand cleaning fluid after the hand cleaning fluid has contacted the user's hand, the fluid analysis mechanism comprising: a fluid collector for collecting at least some of the hand cleaning fluid after the hand cleaning fluid has contacted the user's hand; an analysis chamber that is fluidly connected to the fluid collector for receiving at least some of the hand cleaning fluid collected by the fluid collector; and a camera that captures at least one image of the hand cleaning fluid received by the analysis chamber; wherein the system is configured to detect particles in the at least one image; wherein the system is configured to categorize the particles into predetermined classes; wherein the predetermined classes comprise at least one recognized class and at least one unrecognized class; wherein the system is configured to categorize the particles that meet predetermined criteria into the at least one recognized class; and wherein the system is configured to categorize the particles that fail to meet the predetermined criteria into the at least one unrecognized class.
253. A system, which optionally includes one or more features of any one or more of the previous aspects, wherein the system comprises a machine learning algorithm that is configured to select the predetermined criteria based on training data; and wherein the training data comprises images of the hand cleaning fluid captured by the camera during a training operation.
254. A system, which optionally includes one or more features of any one or more of the previous aspects, wherein the system is configured to determine a background rate of the particles that are categorized into each of the at least one recognized class and each of the at least one unrecognized class based at least in part on images of the hand cleaning fluid captured by the camera over time; and wherein the system is configured to detect the infection based at least in part on detection of an increase in a quantity of the particles that are categorized into at least one of the at least one recognized class and the at least one unrecognized class by at least a threshold amount above the background rate.
255. A system, which optionally includes one or more features of any one or more of the previous aspects, wherein the predetermined criteria comprise at least one of: a size of the particles; a shape of the particles; a movement of the particles; and a cluster characteristic of the particles.
256. A system, which optionally includes one or more features of any one or more of the previous aspects, wherein the camera is configured to capture a series of images of the hand cleaning fluid received by the analysis chamber; wherein the fluid analysis mechanism comprises a stop mechanism that stops a flow of the hand cleaning fluid through the analysis chamber; wherein at least some of the series of images are captured after the flow of the hand cleaning fluid through the analysis chamber has stopped; and wherein the system is configured to identify the particles as active or inactive based at least in part on the movement of the particles over time after the flow of the hand cleaning fluid through the analysis chamber has stopped.
257. A system, which optionally includes one or more features of any one or more of the previous aspects, wherein the system is configured to calculate a sanitation effectiveness value based at least in part on a comparison of a quantity of the particles that are identified as active and a quantity of the particles that are identified as inactive; wherein the system is configured to modify an operation of the fluid dispenser if the sanitation effectiveness value falls below a predetermined standard; and wherein the modification comprises at least one of: changing a formulation of the hand cleaning fluid that is dispensed; and changing a volume of the hand cleaning fluid that is dispensed.
258. A system, which optionally includes one or more features of any one or more of the previous aspects, wherein the analysis chamber comprises an analysis cone; wherein the analysis cone has an apex, a base, and an internal cavity; wherein the internal cavity is in fluid communication with a fluid inlet located at the apex; wherein the internal cavity is in fluid communication with a plurality of fluid outlets located around a periphery of the base; wherein a height of the internal cavity decreases as the internal cavity extends radially outwardly from the apex; wherein the internal cavity is defined between an upper surface and a lower surface; wherein a distance between the upper surface and the lower surface decreases as the internal cavity extends radially outwardly from the apex; and wherein the predetermined criteria comprise at least one of: a location where the particles become lodged between the upper surface and the lower surface; and a distance that the particles drift after the flow is stopped.
259. A system, which optionally includes one or more features of any one or more of the previous aspects, wherein the stop mechanism is configured to equalize a fluid pressure upstream of the analysis chamber and downstream of the analysis chamber, so as to stop the flow of the hand cleaning fluid through the analysis chamber; wherein the stop mechanism comprises an upstream valve, a downstream valve, and an equalization conduit; wherein the upstream valve is located upstream of the analysis chamber; wherein the downstream valve is located downstream of the analysis chamber; wherein the upstream valve has a flow position, in which the analysis chamber is fluidly disconnected from the equalization conduit via the upstream valve; wherein the downstream valve has a flow position, in which the analysis chamber is fluidly disconnected from the equalization conduit via the downstream valve; wherein the upstream valve has a stop position, in which the analysis chamber is in fluid communication with the equalization conduit via the upstream valve; and wherein the downstream valve has a stop position, in which the analysis chamber is in fluid communication with the equalization conduit via the downstream valve.

### Brief Description of the Drawings

Further aspects and advantages of the invention will appear from the following description taken together with the accompanying drawings, in which:
Figure 1 is a perspective view of a fluid dispenser in accordance with a first embodiment of the present invention;
Figure 2 is a front view of the fluid dispenser shown in Figure 1;
Figure 3 is a front view of the fluid dispenser shown in Figure 1, showing a user's hand placed under a fluid outlet of the dispenser;
Figure 4 is a front view of the fluid dispenser shown in Figure 1, showing fluid being dispensed onto the user's hand;
Figure 5 is a schematic front view of a fluid analysis mechanism of the fluid dispenser shown in Figure 1, including a pump, an analysis chamber, and a camera;
Figure 6 is a schematic front view of a first alternative structure for the fluid analysis mechanism shown in Figure 5;
Figure 7 is a schematic front view of a second alternative structure for the fluid analysis mechanism shown in Figure 5;
Figure 8 is a simplified illustration of an image captured by a camera of the fluid analysis mechanism shown in Figure 5, showing particles of different sizes and shapes;
Figure 9 is a simplified illustration of a side view composite image showing movement of particles over time captured by the camera shown in Figure 5;
Figure 10 is a simplified illustration of a top view composite image showing movement of particles over time captured by the camera shown in Figure 5;
Figure 11 is a side view of an alternative structure for the analysis chamber shown in Figure 5;
Figure 12 is a cross-sectional side view of the analysis chamber shown in Figure 11;
Figure 13 is a simplified illustration of a bottom view composite image showing movement of particles over time in the analysis chamber shown in Figure 11;
Figure 14 is a schematic representation of a system including a plurality of the fluid dispensers shown in Figure 1;
Figure 15 is a schematic representation of an alternative system including a plurality of the fluid dispensers shown in Figure 1 and a server;
Figure 16 is a schematic top view of a city showing the locations of the fluid dispensers of the system shown in Figure 15;
Figure 17 is a schematic top view of the city shown in Figure 16, showing a state of the fluid dispensers on day 0 of an outbreak;
Figure 18 is a schematic top view of the city shown in Figure 16, showing the state of the fluid dispensers on day 1 of the outbreak;
Figure 19 is a schematic top view of the city shown in Figure 16, showing the state of the fluid dispensers on day 2 of the outbreak;
Figure 20 is a schematic top view of the city shown in Figure 16, showing the state of the fluid dispensers on day 3 of the outbreak;
Figure 21 is a diagram showing a possible structure for an artificial intelligence classifier of the fluid dispenser shown in Figure 1; and
Figure 22 is a diagram showing a possible structure for an artificial intelligence identifier of the fluid dispenser shown in Figure 1.

### Detailed Description of the Drawings

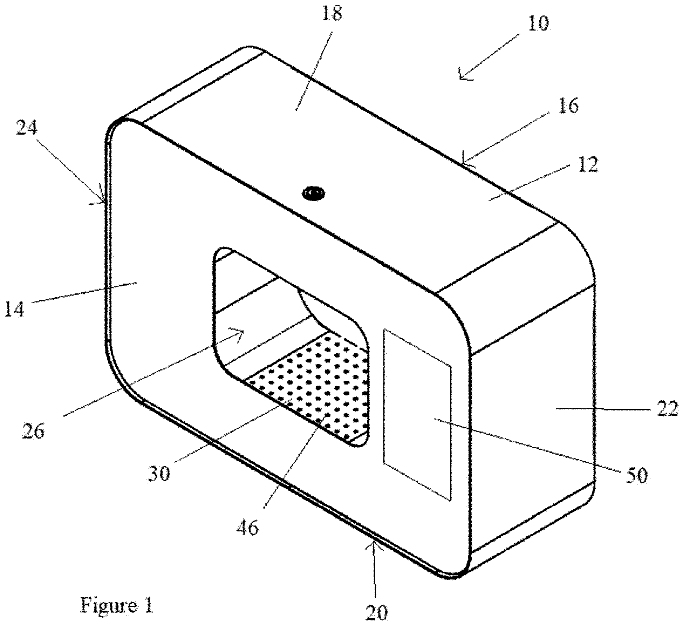

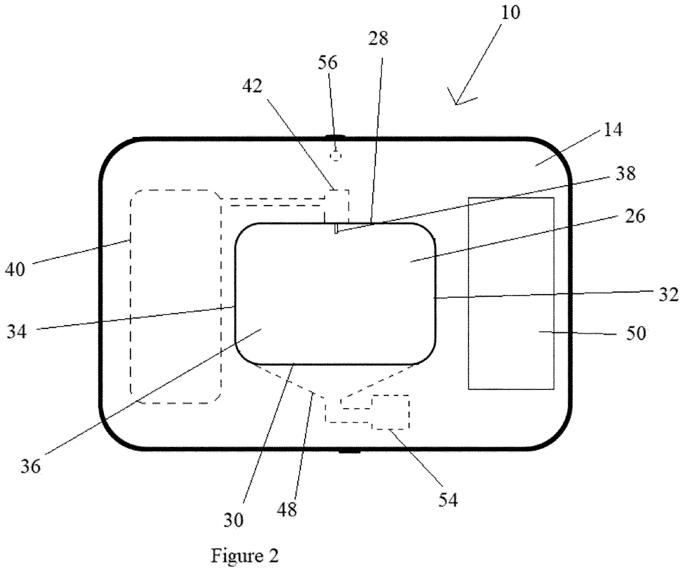

Figures 1 and 2 show a hand cleaning fluid dispenser 10 in accordance with a first embodiment of the present invention. The dispenser 10 has a generally rectangular body 12 with a front wall 14, a back wall 16, a top wall 18, a bottom wall 20, a right side wall 22, and a left side wall 24. The body 12 has a forwardly open chamber 26 that extends rearwardly into the body 12 from the center of the front wall 14. The chamber 26 is defined by an upper wall 28, a lower wall 30, a right lateral wall 32, a left lateral wall 34, and a rear wall 36.

The body 12 contains at least one fluid reservoir 40 and at least one pump mechanism 42, as shown in dotted lines in Figure 2. The fluid reservoir 40 contains a supply of a hand cleaning fluid 44, such as soap or hand sanitizer. The pump mechanism 42 is configured to dispense the fluid 44 from the fluid outlet 38 when the pump mechanism 42 is activated. Any suitable construction for the fluid reservoir 40 and the pump mechanism 42 could be used, including for example those disclosed in United States Patent No. 9,437,103 to Ophardt, issued September 6, 2016, and United States Patent Application Publication No. 2022/0091011 to Steltenkamp et al., published March 24, 2022, which are incorporated herein by reference.

As can be seen in Figure 1, the lower wall 30 of the chamber 26 has a plurality of apertures 46. The apertures 46 allow fluid 44 that falls onto the lower wall 30 to pass through the lower wall 30 and into a fluid collector 48 that is positioned below the lower wall 30. The fluid collector 48 is shown schematically in Figure 2 by dotted lines. The fluid collector 48 delivers the fluid 44 to a fluid analysis mechanism 54, also shown schematically in Figure 2 by dotted lines.

The fluid analysis mechanism 54 is configured to analyze the fluid 44 to detect particles 68 or other contaminants present therein. In preferred embodiments, the fluid analysis mechanism 54 is configured to detect biological particles, such as viruses, bacteria, spores, yeast, and/or fungi. The fluid analysis mechanism 54 may have any structure suitable for analyzing the fluid 44 in order to detect materials, components, particles, and/or contaminants present therein. The fluid analysis mechanism 54 may, for example, have one or more of the structures disclosed in United States Patent No. 9,437,103 to Ophardt, issued September 6, 2016, and United States Patent Application Publication No. 2022/0091011 to Steltenkamp et al., published March 24, 2022.

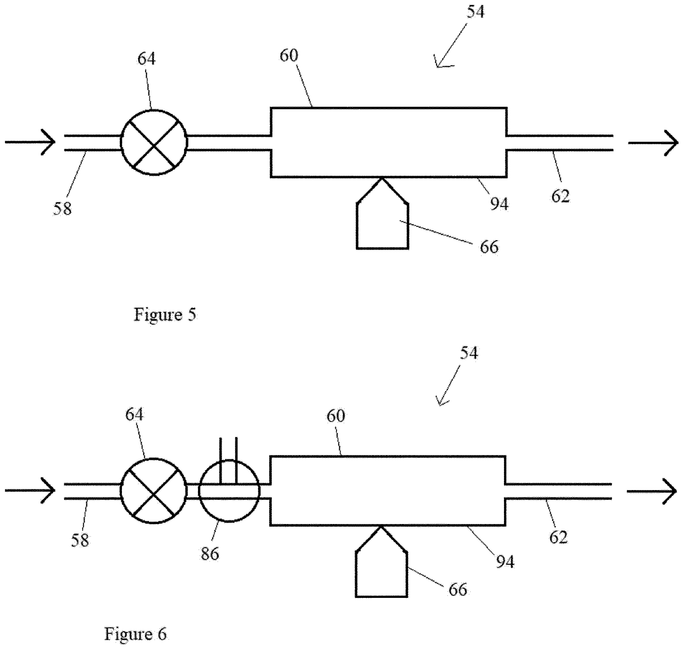

One possible structure for the fluid analysis mechanism 54 is shown schematically in Figure 5. The fluid analysis mechanism 54 shown in Figure 5 includes a fluid inlet channel 58, a fluid analysis chamber 60, a fluid outlet channel 62, a fluid pump 64, and an electromagnetic detection device, for example a camera 66. The fluid pump 64 draws fluid 44 collected by the fluid collector 48 through the fluid inlet channel 58 and into the fluid analysis chamber 60 for analysis. The camera 66 is configured to capture at least one image, and preferably a series of images, of the fluid 44 in the fluid analysis chamber 60.

After the desired images of the fluid 44 in the fluid analysis chamber 60 have been captured, the fluid 44 is pumped out of the fluid analysis chamber 60 through the fluid outlet channel 62 by the fluid pump 64. The fluid outlet channel 62 may, for example, flow into a waste storage container, not shown. It is to be appreciated that the fluid pump 64 could be located at any position in the flow path of the fluid 44, and need not be in the location shown in Figure 5. Multiple fluid pumps 64 could also be provided.

As can be seen in Figure 2, the front wall 14 of the fluid dispenser 10 has a display 50, which is positioned to the right of the chamber 26. The display 50 may be configured to display any desired information, text, pictures, and/or graphics. For example, the display 50 may be configured to provide visual, video, and/or audiovisual prompts and instructions to assist a user in the operation of the fluid dispenser 10. The display 50 can also be configured to display information such as the results of the fluid analysis, the type of fluid 44 being dispensed, the quantity of fluid 44 being dispensed, and the quantity of fluid 44 remaining in the fluid reservoir 40. Optionally, the display 50 is a touchscreen that can be touched in order to input commands and information. For example, various settings of the fluid dispenser 10 may be selected or adjusted using the touchscreen display 50, such as the quantity of fluid 44 dispensed with each activation.

Optionally, the fluid dispenser 10 may include a user-facing camera 56, shown in dotted lines in Figure 2. Although not shown in the drawings, the fluid dispenser 10 preferably also has one or more hand sensors that are able to detect the presence of a hand 52 inserted into the chamber 26. Any suitable type of sensor capable of detecting the presence of a hand 52 could be used, such as an infrared sensor or a time of flight sensor. The sensor or sensors may be placed at any suitable location for detecting the presence of a hand 52, such as on the upper wall 28, the lateral walls 32, 34, or the rear wall 36 of the chamber 26.

Although not shown in the drawings, the fluid dispenser 10 also preferably includes at least one processor that processes data received from the fluid analysis mechanism 54, the hand sensor, the display 50, and/or the user-facing camera 56, and controls the functioning of the fluid analysis mechanism 54, the display 50, the pump mechanism 42, and/or the user-facing camera 56. The fluid dispenser 10 also includes at least one communication device, not shown, that allows the dispenser 10 to communicate with other devices, such as through Wi-Fi, Bluetooth, Near-Field Communication, or the like. The fluid dispenser 10 also preferably includes other electronic components such as at least one memory and at least one power source, such as a battery.

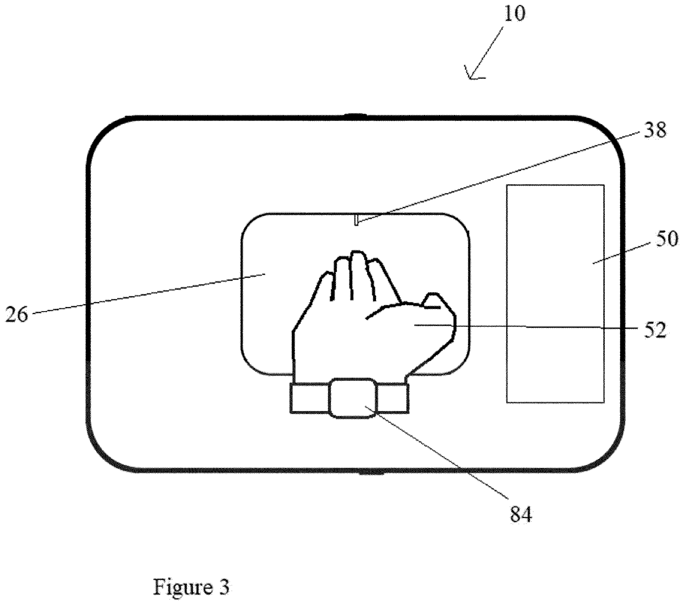

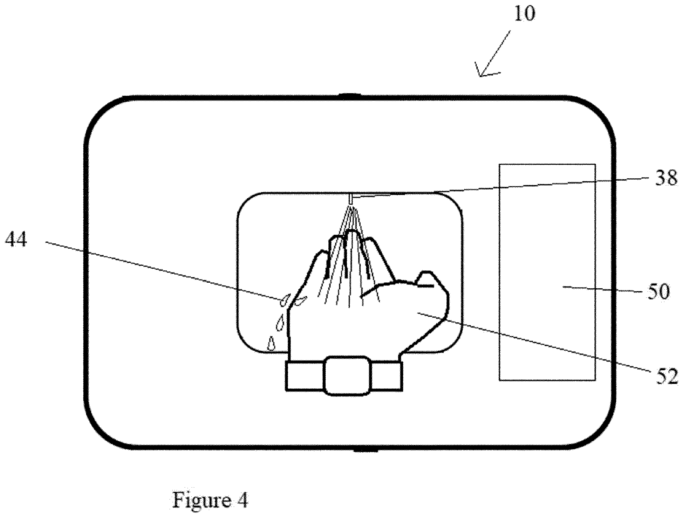

One preferred manner of operating the fluid dispenser 10 will now be described with reference to the Figures. As shown in Figure 3, to operate the fluid dispenser 10 a user places their hand 52 into the chamber 26, below the fluid outlet 38. The presence of the user's hand 52 in the chamber 26 is detected by the hand sensor, which causes the pump mechanism 42 to activate and dispense fluid 44 onto the user's hand 52, as shown in Figure 4. Preferably, the fluid dispenser 10 is configured to provide a volume and/or pressure and/or spray pattern of the dispensed fluid 44 that causes at least some of the fluid 44 to contact the user's hand 52 and then drip off the user's hand 52 and into the fluid collector 48. After contacting the user's hand 52, the fluid 44 preferably carries a sample of the particles 68 that are present on the user's hand 52.

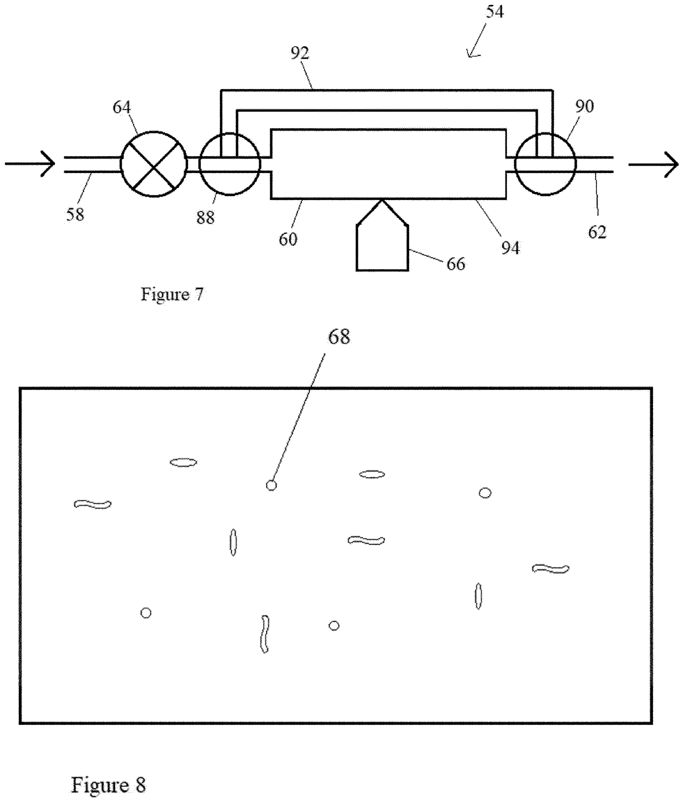

The fluid 44 that has fallen off of the user's hand 52 is collected in the fluid collector 48, and drawn into the fluid analysis chamber 60 by the fluid pump 64. An electromagnetic detection device, for example an optical camera 66 captures at least one data set of electromagnetic information of the fluid 44, for example an image of the fluid 44. An example of an image that might be captured by the camera 66 is shown in Figure 8. Preferably, the camera 66 is provided with a microscope or a microscopic lens, in order to obtain a magnified image of the fluid 44 in which microscopic particles 68 such as bacteria are visible.

The fluid dispenser 10 is preferably configured to analyze the image or images captured by the camera 66 in order to detect whether the user of the fluid dispenser 10 might have an infection. To assist in detecting an infection, in one preferred embodiment of the invention the fluid dispenser 10 is configured to identify particles 68 in the image or images captured by the camera 66, and categorize each of the particles 68 into predetermined classes.

Preferably, each of the predetermined classes are defined by one or more parameters or characteristics of the particles 68, such as size and/or shape. Optionally, the characteristics defining each of the classes may be set by an administrator or user. More preferably, the fluid dispenser 10 is configured to independently select and/or adjust the criteria defining each of the classes with little or no input from an administrator or user.

For example, in one optional implementation of the invention the fluid dispenser 10 is configured to perform a training operation in which the fluid dispenser 10 analyzes training data in order to independently select criteria defining a set of classes. The training data may, for example, be a series of images of particles 68. Preferably, the training data images are images captured by the fluid dispenser 10 itself during normal operation of the fluid dispenser 10 during the training operation.

The fluid dispenser 10 is preferably configured to identify the particles 68 in the training data images, and to select defining criteria for the classes that will allow similar particles in the training data images to be categorized together in the same class. For example, the fluid dispenser 10 may be configured to select criteria to define the classes so as to minimize variability within the classes.

As a simplified example, the training data may include particles (a) to (j) having the following sizes: (a) 0.5 microns; (b) 1.5 microns; (c) 1.4 microns; (d) 0.6 microns; (e) 3.2 microns; (f) 1.3 microns; (g) 0.6 microns; (h) 3.3 microns; (i) 3.4 microns; and (j) 0.5 microns. In order to minimize variability within the classes, the fluid dispenser 10 may select the criteria for defining three classes of particles (1) to (3) as follows: (1) particles having a size from 0.5 microns to 0.6 microns (particles (a), (d), (g), and (j); (2) particles having a size from 1.3 microns to 1.5 microns (particles (b), (c), and (f)); and (3) particles having a size from 3.2 microns to 3.4 microns (particles (e), (h), and (i)).

Optionally, the fluid dispenser 10 may be configured to select the number of classes, or to select the number of classes within a range (e.g. between 3 and 10 classes). Alternatively, the number of classes may be predetermined.

Preferably, the fluid dispenser 10 is configured to have at least one recognized class and at least one unrecognized class. The at least one recognized class includes all of the classes for which the fluid dispenser 10 has selected defining characteristics. Particles that do not meet the criteria for any of the recognized classes are categorized into the at least one unrecognized class. This ensures that the fluid dispenser 10 is able to detect and quantify new particle types that were not present in the training data.

The training operation may continue for any desired length of time, such as one day, one week, or one month. In preferred embodiments, the training operation continues indefinitely, with the fluid dispenser 10 continuously or periodically updating the criteria defining the recognized classes based on the data received from the fluid analysis mechanism 54 over time. The fluid dispenser 10 may, for example, include a machine learning algorithm that continuously or periodically monitors and assesses the effectiveness of the classification criteria in (1) classifying as many of the detected particles 68 as possible into recognized classes while (2) minimizing the variability within the classes. The machine learning algorithm preferably updates the classification criteria periodically in order to improve performance over time.

In order to detect the possible presence of an infection, the fluid dispenser 10 is preferably configured to compare the quantity of particles 68 in each class that are detected in a sample of the fluid 44 to a background rate. The background rate is preferably determined by the fluid dispenser 10 based on historical data. For example, the fluid dispenser 10 may be configured to quantify the number of particles 68 that are categorized into each of the classes each time that an image of the fluid 44 is captured by the camera 66. The background rate for each class may, for example, be calculated as the average (or mean) quantity of particles 68 categorized in that class per image captured.

The fluid dispenser 10 is preferably configured to determine that an infection has been detected if the quantity of particles 68 exceeds the background rate, preferably by some predetermined threshold. For example, the fluid dispenser 10 may be configured to calculate a standard deviation based on the historical data, and the predetermined threshold may be defined as a quantity of particles 68 in a class that exceeds the background rate by at least one standard deviation. Any other suitable criteria for setting the predetermined threshold could also be used. The fluid dispenser 10 may also optionally use a machine learning algorithm to evaluate and update the predetermined threshold over time.

If the fluid dispenser 10 determines that the quantity of particles 68 detected in a sample of the fluid 44 exceeds the background rate by the predetermined threshold in one or more classes, the fluid dispenser 10 preferably determines that the user from which the sample was obtained has an infection or a possible infection. Upon detecting an infection, the fluid dispenser 10 is preferably configured to modify an operation of the fluid dispenser 10.

For example, the fluid dispenser 10 may be configured to provide a notification when a possible infection has been detected, such as by providing a visual and/or audiovisual alert on the display 50. Alerting the user that they possibly have an infection preferably encourages the user to take measures to decrease the risk of transmitting the infection to others, such as by wearing a mask, thoroughly sanitizing their hands, avoiding contact with vulnerable individuals, and/or self-isolating. Optionally, the fluid dispenser 10 may instruct the user to take certain steps to decrease the risk of transmitting the infection. The fluid dispenser 10 may also instruct the user to seek out further medical testing, such as to verify the presence of an infection and/or to identify the type of infection.

Advantageously, the fluid dispenser 10 is not limited to detecting a narrow set of previously known infectious particles 68. Rather, new particles 68 that meet the criteria of any of the recognized classes will be quantified, with an unusual spike in the quantity triggering the detection of an infection. Furthermore, if the new particles 68 do not meet the criteria of any of the recognized classes, the new particles 68 will be quantified in the at least one unrecognized class. An unusual spike in unrecognized particles 68 will also trigger the detection of an infection. The fluid dispenser 10 is thus preferably able to detect the presence of an infection caused by a novel agent, such as a previously unknown bacteria or parasite.

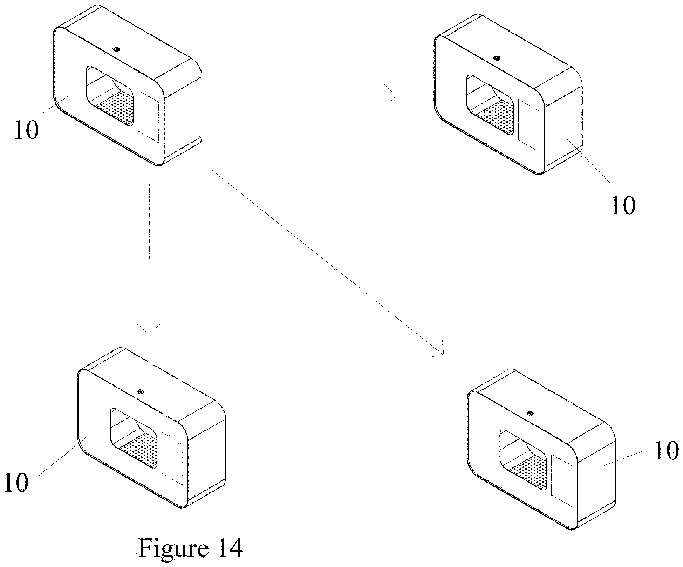

Preferably, the fluid dispenser 10 is one of a plurality of fluid dispensers 10 that are communicatively linked to each other to form an interconnected system, an example of which is shown schematically in Figure 14. By communicating with each other, such as via Wi-Fi or the like, the fluid dispensers 10 are preferably able to use swarm intelligence to provide a dynamic and rapid response to the presence of an infection.

As a simplified example, four fluid dispensers 10 could be arranged at different locations in a building. When one of the fluid dispensers 10 detects an infection, this information is preferably communicated to the other fluid dispensers 10 in the environment (shown by the arrows in Figure 14). The other fluid dispensers 10 are able to use this information to rapidly respond to the possible presence of an infection, even before the infection has been directly detected by those dispensers 10.

For example, upon receiving information that one of the fluid dispensers 10 has detected an infection, the remaining fluid dispensers 10 could display a notification on their respective displays 50 indicating that an infection has been detected in the building, and encouraging thorough hand cleaning. The fluid dispensers 10 could also be configured to take other steps when an infection is detected by one of the dispensers 10, such as altering the volume and/or formulation of the fluid 44 that is dispensed, in order to increase the efficacy of the fluid 44 in sanitizing user's hands 52.

In one optional embodiment, the fluid analysis mechanisms 54 of one or more of the fluid dispensers 10 in the environment may be deactivated as a default condition, in order to reduce waste and delay maintenance. Preferably, at least one of the fluid dispensers 10 in the environment acts as a sentinel fluid dispenser 10, with its fluid analysis mechanism 54 active in order to detect possible infections in the environment. Upon detecting an infection, the sentinel fluid dispenser 10 preferably communicates this information to the remaining fluid dispensers 10 in the environment. In response, the fluid analysis mechanisms 54 of the remaining fluid dispensers 10 are preferably activated, allowing the system to collect further information about the spread of the infection.

Preferably, the system is configured so that the fluid dispensers 10 respond to the detection of an infection in different ways depending on their location relative to the location where the infection was detected. For example, the environment in which the fluid dispensers 10 are located could be divided into different zones, and the response of the fluid dispensers 10 could depend on which zone they are located in. For example, if a fluid dispenser 10 in one zone detects an infection, all of the other fluid dispensers 10 in that zone could react by activating their respective fluid analysis mechanisms 54, changing the volume and/or formulation of the fluid 44 being dispensed, and providing notifications encouraging thorough hand cleaning on their respective displays 50. The fluid dispensers 10 in adjacent zones could respond by activating their respective fluid analysis mechanisms 54 and providing notifications encouraging thorough hand cleaning on their respective displays 50, without changing the volume and/or formulation of the fluid 44 being dispensed. The fluid dispensers 10 in zones that are further away may not respond to the detection of the infection at all. The system is thus preferably able to provide a response to an infection that can react in an intelligent and localized way, and that can adapt intelligently as an infection spreads through an environment.

In some embodiments of the invention, the system is configured to notify an authority such as administrator of the system, an administrator of a building, a public health authority, or the like when an infection is detected, so that the authority can take steps to investigate the infection and reduce the risk of the infection spreading.

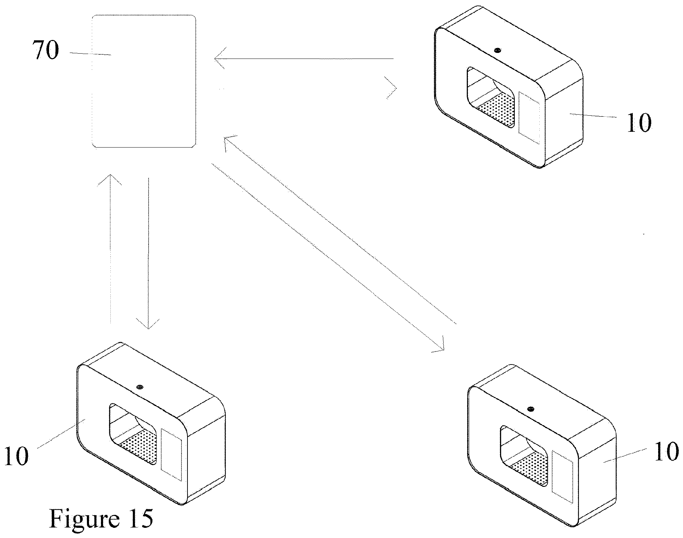

In some embodiments of the invention, the system includes at least one server 70 or central communication unit in addition to the fluid dispensers 10, as shown in Figure 15. The server 70 is preferably configured to engage in two-way communication with each of the fluid dispensers 70, as shown by the arrows in Figure 15. The server 70 is preferably configured to collect data from the fluid dispensers 10 and coordinate the response of the system to the detection and spread of an infection.

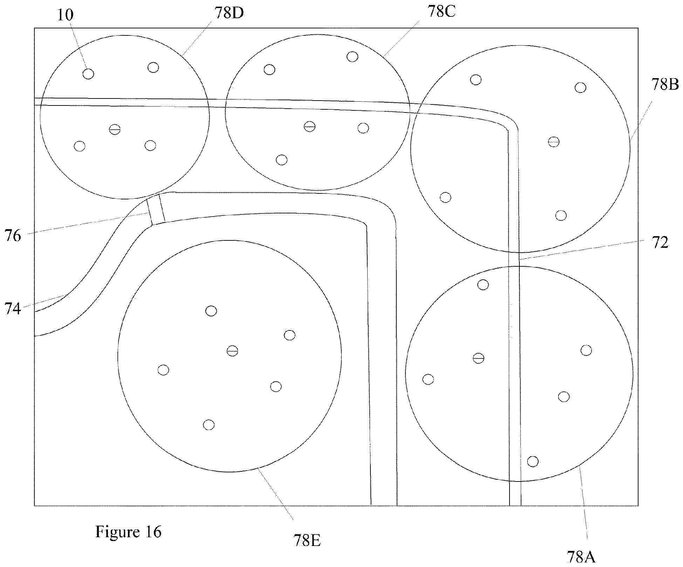

An example of the manner in which the system could be configured to respond to the spread of an infection is shown in Figures 16 to 20. Figure 16 shows a schematic top view of a city with a transit line 72, a river 74, and a bridge 76. A plurality of fluid dispensers 10 are positioned at different locations around the city. The fluid dispensers 10 are located in five different zones 78A, 78B, 78C, 78D, 78E.

Figure 16 shows the status of the fluid dispensers 10 under default conditions, when no infections have been detected. As can be seen in Figure 16, each of the zones 78A, 78B, 78C, 78D, 78E has at least one sentinel fluid dispenser 10, in which the fluid analysis mechanism 54 is active. The fluid dispensers 10 in which the fluid analysis mechanism 54 is active are denoted in the Figures as a circle with a horizontal line. In the remaining fluid dispensers 10, denoted by a circle without a horizontal line, the fluid analysis mechanisms 54 are inactivated in order to save resources and reduce maintenance requirements.

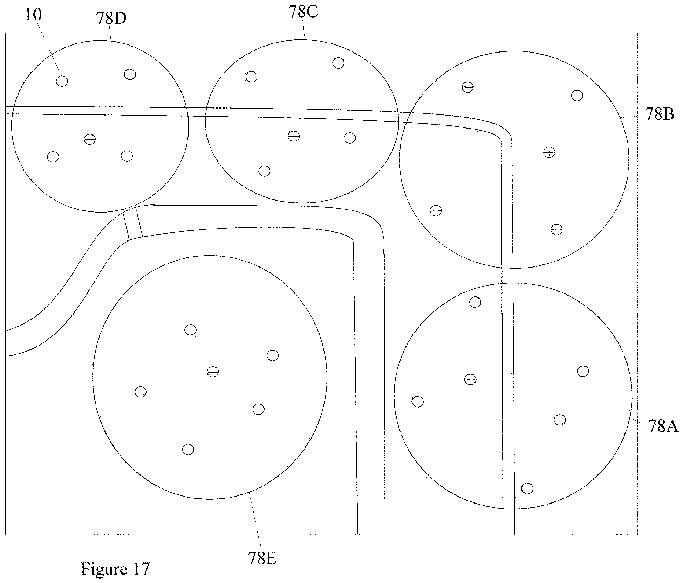

Figure 17 shows the status of the fluid dispensers 10 on the first day that an infection is detected by one of the sentinel fluid dispensers 10, which is also referred to as day 0 of the outbreak. As can be seen in Figure 17, the sentinel fluid dispenser 10 that has detected the infection is located in zone 78B. A fluid dispenser 10 that has detected an infection is denoted in the Figures by a circle having both a horizontal line and a vertical line.

Upon detecting an infection, the sentinel fluid dispenser 10 communicates this information to the server 70. In response, the server 70 instructs the other fluid dispensers 10 in zone 78B to activate their respective fluid analysis mechanisms 54.

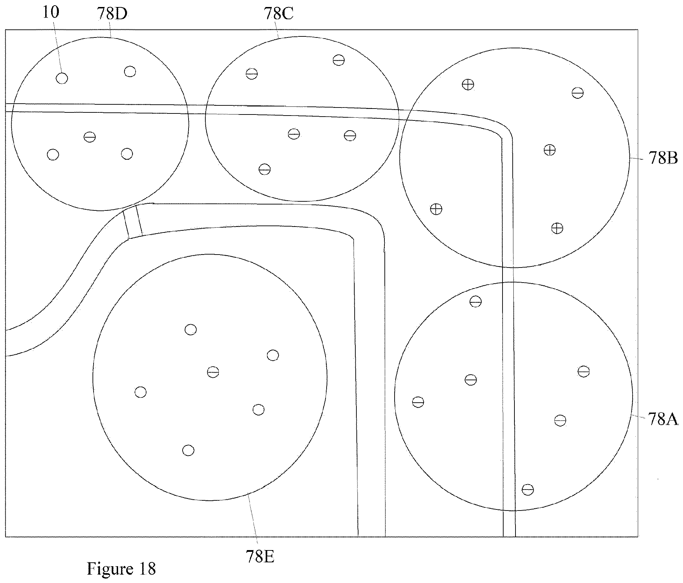

Figure 18 shows the status of the fluid dispensers 10 on the following day, which is also referred to as day 1. On day 1, several of the other fluid dispensers 10 in zone 78B have detected an infection, and this information is communicated to the server 70. In response, the server 70 instructs all of the fluid dispensers 10 in the adjacent zones 78A and 78C to activate their respective fluid analysis mechanisms 54. The server 70 may also take other actions, such as alerting a public health authority that an infection appears to be spreading in zone 78B. The server 70 may also instruct the fluid dispensers 10 in zones 78A, 78B, and 78C to provide notifications that an infection appears to be spreading in the area, and to convey other public health information and/or instructions.

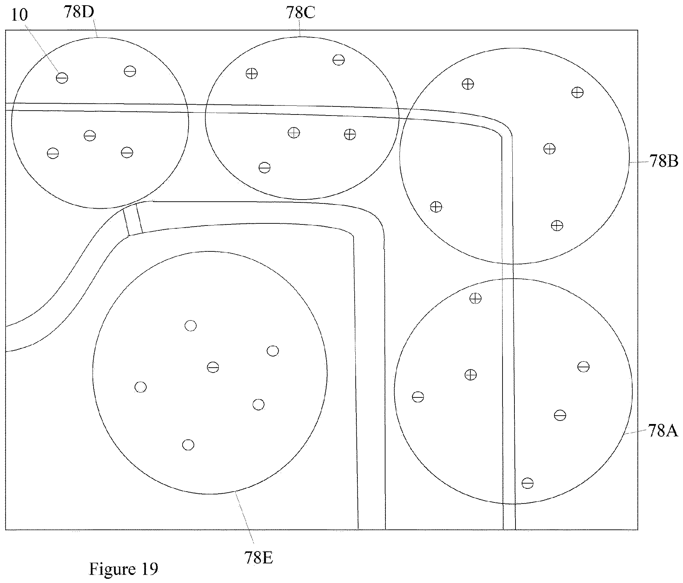

Figure 19 shows the status of the fluid dispensers 10 on the following day, which is also referred to as day 2. On day 2, several of the fluid dispensers 10 in zones 78A and 78C have detected an infection, and this information is communicated to the server 70. In response, the server 70 instructs the fluid dispensers 10 in zone 78D to activate their fluid analysis mechanisms 54. The server 70 may also take other actions, such as advising a public health authority that the infection appears to now be spreading in zones 78A, 78B, and 78C.

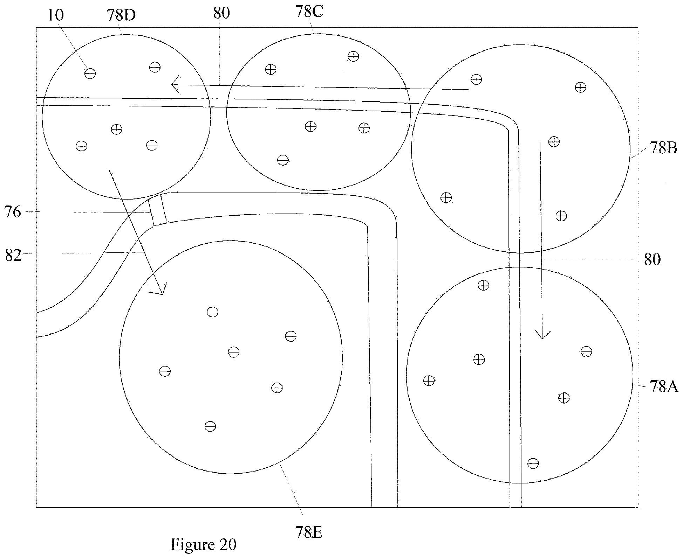

Figure 20 shows the status of the fluid dispensers 10 on the following day, which is also referred to as day 3. On day 3, one of the fluid dispensers 10 in zone 78D has detected an infection. In response, the server 70 instructs the fluid dispensers 10 in zone 78E to activate their fluid analysis mechanisms 54.

The server 70 is preferably configured to record information about the location where infections were detected over time. This information may be used, for example, to determine a path that an infection has spread through an environment. For example, based on the data collected on days 0 to 3 as shown in Figures 17 to 20, the server 70 is able to determine that the infection appears to have originated in zone 78B, and rapidly spread to multiple locations in zone 78B within about 1 day. The infection then appears to have spread to the adjacent zones 78A and 78C about one day later. From zone 78C, the infection then appears to have spread to zone 78D on the following day.

Based on this information, the server 70 is able to reconstruct the path or paths that the infection has spread through the city over time, shown by the arrows 80 in Figure 20. This information is preferably recorded, and can be used to improve the performance of the system over time. The information may also be shared with public health authorities, in order to help guide and inform public health decisions.

In some preferred embodiments, the server 70 is configured to predict where the infection will spread in the future. For example, the server 70 may take into account information such as the path that an infection has already spread through the environment, the known geographical details of the environment, the location of borders, the known or predicted movement patterns of people within the environment, and the paths that previous infections have spread through the environment. The server 70 may rely on information collected by the system itself, and optionally may also receive external information such as traffic data, transit data, washroom usage data, event schedule and location data, and hospital admission data from external sources such as technology companies, businesses, governments, hospitals, and health care facilities. Based on this data, the server 70 is preferably able to provide a prediction of where the infection may spread to in the future.

In the example shown in Figure 20, the predicted future path of the infection is shown by the arrow 82. In particular, the server 70 predicts that the infection will spread next to zone 78E. Based on this information, the server 70 is preferably configured to instruct the fluid dispensers 10 in zone 78E to activate their fluid analysis mechanisms 54, and to take other actions such as provide visual notifications encouraging hand cleaning, mask wearing, and the like.

The server 70 may also share the predicted path that an infection will spread with various authorities, such as public health authorities, governments, and/or hospital and long term care facility administrators. This may assist the authorities in making public health decisions to limit the spread of the infection. For example, based on the predicted path 82 shown in Figure 20, public health authorities may seek to limit travel across the bridge 76 or to add a screening requirement for crossing the bridge 76.

In some embodiments of the invention, the system is configured to determine the identity of a user who is suspected of having an infection. For example, the fluid dispensers 10 may be configured to communicate with a device worn or carried by the user, such as an ID card, a smart phone, or a smart watch 84 as shown in Figure 3. The device preferably provides the fluid dispenser 10 with information about the identity of the user.

If the fluid dispenser 10 detects the presence of a possible infection from the sample collected from the user, the fluid dispenser 10 preferably notifies the user. The fluid dispenser 10 may, for example, send a message to the smart watch 84 worn by the user. The fluid dispenser 10 may also provide the identity of the user to an authority, such as a public health authority, an administrator of the system, or an administrator of the building, for example to facilitate contact tracing, or the implementation of public health measures.

Optionally, the system may be configured to record the identity of every user of the fluid dispensers 10. This information can then be used to retrace the movements of a user who is later found to have an infection. This information may be helpful for contact tracing, for determining the path that an infection has spread through an environment, and/or for predicting the future spread of an infection. Alternatively, the system may be configured to determine the identity of a user only when an infection is detected.

Optionally, the system may be configured to collect additional information from a device worn by the user, such as a smart watch 84. For example, the fluid dispensers 10 may be configured to collect physiological data recorded by the smart watch 84, such as body temperature, blood oxygen levels, and heart rate. The system may be configured to take this physiological data into account when determining whether or not the user has an infection.

In some embodiments of the invention, the system may use the user-facing camera 56 to capture an image of the user's face. The image of the user's face can optionally be used for facial recognition, in order to identify the user.

In some embodiments of the invention, the system may be configured to collect and record information about the microbiome of some or all of the users from which samples are collected and analyzed. This information may be used to uniquely identify different users of the system, based on the unique pattern of microbes that are found on their skin. This microbiome data may be used to track the movements of different users of the fluid dispensers 10, in order to assist with contact tracing, for example. Changes in the skin microbiome of an individual may also be used as an indication of a possible infection.

The fluid dispensers 10 in the system may use any structure or structures for their fluid analysis mechanisms 54 that are suitable for the desired analysis, and the fluid dispensers 10 may optionally be provided with fluid analysis mechanisms 54 that differ from each other. In some preferred embodiments of the invention, the fluid analysis mechanisms 54 are configured to perform a stopped-flow analysis.

In the structure shown in Figure 5, the stopped-flow analysis can be performed by pumping the fluid 44 into the analysis chamber 60, and then shutting off the fluid pump 64. A series of images are then captured by any electromagnetic detection methods, for example a camera 66, which are analyzed in order to determine the movements of the particles 68 over time. The camera 66 may, for example, be configured to capture an image every second for 10 seconds, or to capture an image every 5 seconds or every 10 seconds.

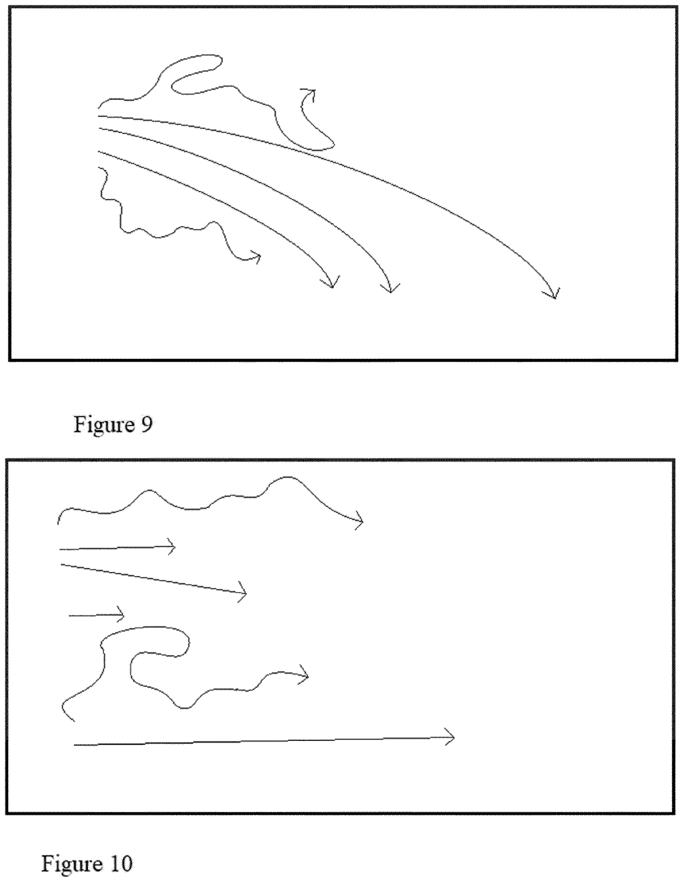

A simplified illustration of a composite image showing a side view of the movement of five particles 68 during a stopped-flow analysis is shown in Figure 9. As can be seen in Figure 9, three of the particles 68 drift forward and downwards in an arc, and two of the particles 68 exhibit a random-walk type of movement in various directions. The inventors have appreciated that inactive or dead particles 68 will usually drift forwards for a short distance, in the direction of the previous fluid flow, and downwards to form a sediment over time. The inventors have further appreciated that active or live particles 68 will often exhibit a random-walk type of movement, caused by the movement of a live bacteria's flagella. Preferably, the movement of the particles 68 is detected in three dimensions, including speed and acceleration as a three dimensional vector.

The system is preferably configured to distinguish between live or active particles and dead or inactive particles based on the stopped-flow analysis. This information may be used, for example, to calculate an effectiveness value for the hand sanitizing event from which the sample was obtained. For example, if the fluid analysis mechanism 54 detects any live or active particles 68, the system may be configured to determine that the hand sanitizing event was unsuccessful.

If the fluid dispenser 10 determines that a hand sanitizing event was unsuccessful, the fluid dispenser 10 may provide a notification to the user and/or to a system administrator. The fluid dispenser 10 may also recommend that the user perform further hand cleaning actions, for example by displaying the recommendations on the display 50 and/or on the user's smart watch 84. The fluid dispenser 10 may also take actions to increase the effectiveness of the fluid 44 that is dispensed, for example by increasing the volume of fluid 44 that is dispensed or altering a formulation of the fluid 44.

In some embodiments of the invention, the fluid dispenser 10 may be configured to calculate the effectiveness value in terms of the percentage or fraction of particles 68 that are active versus inactive. The effectiveness value is preferably recorded, and may be used by a machine learning algorithm to improve the effectiveness of the fluid dispensers 10 over time.

Preferably, the system is configured to use the movements of the particles 68 over time as a criteria when classifying the particles 68 into the recognized and unrecognized classes. For example, the distance that a particle 68 drifts before it forms a sediment in the analysis chamber 60 may be a function of the size and/or shape of the particle 68. In particular, larger particles 68 usually stop faster and begin to sediment before smaller particles 68. Furthermore, active or alive bacterial particles 68 of different species may exhibit different patterns of movement, which can be used to categorize the particles into different classes.

The images for the stopped-flow analysis may be taken from any suitable position. For example, in the composite image shown in Figure 10, the images have been captured from above. The dead or inactive particles can be clearly distinguished from the live or active particles in the composite image shown in Figure 10 by the linear movement of the inactive particles compared to the random-walk type movement of the active particles.

Various different structures may be used in order to stop the flow of the fluid 44 for the stopped-flow analysis. For example, in the alternative structure shown in Figure 6, an atmospheric valve 86 is positioned between the fluid pump 64 and the analysis chamber 60. When fluid 44 is being pumped into the analysis chamber 60, the atmospheric valve 86 is in a closed position, in which the valve 86 is closed to the atmosphere. In order to stop the fluid 55 flow through the analysis chamber 60, the atmospheric valve 86 is moved to an open position, in which the valve 86 is open to the atmosphere. This preferably immediately depressurizes the fluid inlet channel 58, stopping the flow of fluid 44 through the chamber 60.

A further alternative structure for the fluid analysis mechanism 54 is shown in Figure 7. In the structure shown in Figure 7, there is an upstream valve 88 positioned upstream of the analysis chamber 60, a downstream valve 90 positioned downstream of the analysis chamber 60, and an equalization conduit 92 that extends between the upstream valve 88 and the downstream valve 90.

The upstream valve 88 and the downstream valve 90 each have a flow position and a stop position. When the upstream valve 88 and the downstream valve 90 are in the flow position, the analysis chamber 60 is fluidly disconnected from the equalization conduit 92. In order to stop the flow of fluid 44 through the analysis chamber 60, the upstream valve 88 and the downstream valve 90 are moved to the stop position. When in the stop position, the upstream valve 88 is fluidly connected to the equalization conduit 92 upstream of the analysis chamber 60, and the downstream valve 90 is fluidly connected to the equalization conduit 92 downstream of the analysis chamber 60. The fluid connection between the upstream valve 88 and the downstream valve 90 allows the fluid pressure upstream of the analysis chamber 60 to rapidly equalize with the fluid pressure downstream of the analysis chamber 60, stopping the flow of fluid 44 through the chamber 60.

The analysis chamber 60 may have any suitable structure that permits the desired analysis to take place. For example, the analysis chamber 60 may be formed as an analysis channel 94, as in the structures shown schematically in Figures 5 to 7. The analysis channel 94 may optionally be larger than the fluid inlet channel 58 and the fluid outlet channel 62; smaller than the fluid inlet channel 58 and the fluid outlet channel 62; or the same size as the fluid inlet channel 58 and the fluid outlet channel 62. Preferably, the analysis chamber 60 has at least one surface that is transparent, so as to allow the camera 66 to capture images of the fluid 44 within the chamber 60.

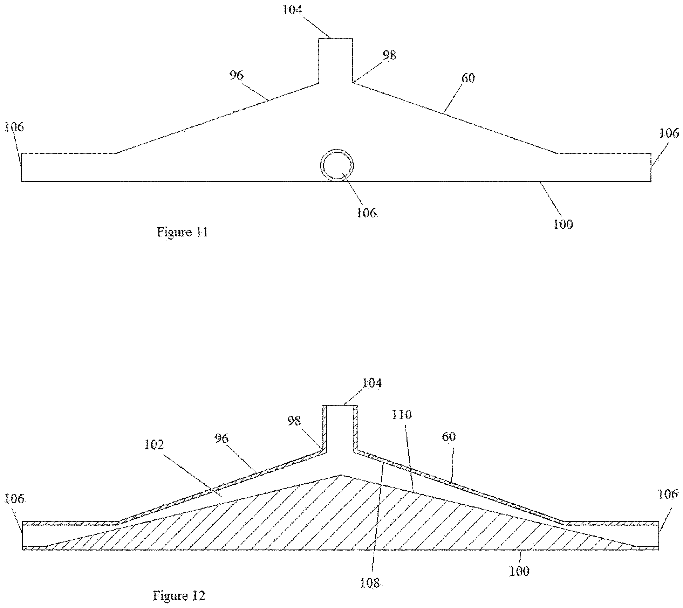

An alternative structure for the analysis chamber 60 is shown in Figures 11 to 13. The analysis chamber 60 shown in Figures 11 to 13 is in the form of an analysis cone 96. The analysis cone 96 has an apex 98, a base 100, and an internal cavity 102. As best shown in Figure 12, the internal cavity 102 is in fluid communication with a fluid inlet 104 located at the apex 98, and with a plurality of fluid outlets 106 located around a periphery of the base 100.

As can be seen in Figure 12, the internal cavity 102 is defined between an upper surface 108 and a lower surface 110. As the internal cavity 102 extends radially outwardly from the apex 98, the height of the internal cavity 102, defined by the distance between the upper surface 108 and the lower surface 110, decreases.

The internal cavity 102 of the analysis cone 96 is configured to receive the fluid 44 collected by the fluid collector 48 via the fluid inlet 104. The fluid 44 passes through the internal cavity 102, and flows out of the analysis cone 96 via the fluid outlets 106.

The camera 66 is preferably configured to capture at least one image of the fluid 44 in the analysis cone 96, and preferably a series of images. Preferably, the flow of fluid 44 through the analysis cone 96 is stopped while the images are being captured. The base 100 of the analysis cone 96 may, for example, be transparent, so as to allow the camera 66 to capture images of the fluid 44 within the internal cavity 102 from below.

The inventors have appreciated that the analysis cone 96 preferably allows the fluid analysis mechanism 54 to collect useful information about the particles 98 that are present in the fluid 44. For example, because the height of the internal cavity 102 preferably decreases as the internal cavity 102 extends radially outwardly from the apex 98, at least some particles 68 may become lodged between the upper surface 108 and the lower surface 110 before reaching the fluid outlets 106. Furthermore, the location where a particle 68 becomes lodged will be a function of the size of the particle 68, with larger particles 68 becoming lodged closer to the apex 98 and smaller particles 68 becoming lodged further from the apex 98.

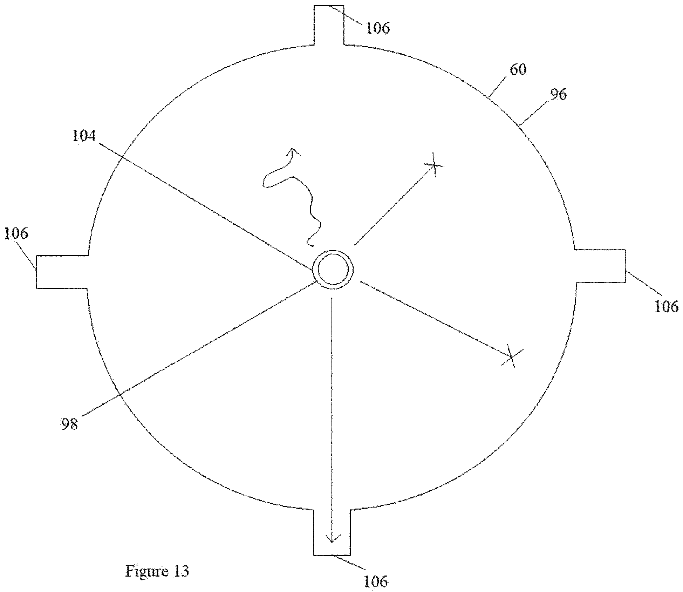

A simplified example of a composite image showing the movements or flow-traces of various particles 68 through the analysis chamber 60 over time is shown in Figure 13. As can be seen in Figure 13, the active or alive particles 68 can be distinguished from the dead or inactive particles 68 based on whether the particles 68 move in a linear fashion or in a random-walk. Furthermore, the relative sizes of the particles 68 can be determined relatively easily, with larger particles 68 becoming lodged (denoted by an X) closer to the apex 98, and with smaller particles 68 becoming lodged further from the apex 98 or flowing out through the fluid outlets 106.

The fluid dispenser 10 is preferably configured to use the position where a particle 68 stops or becomes lodged in the analysis cone 96 as one of the criteria for categorizing the particles 98 into different classes.

Optionally, in order to clean the analysis cone 96 and remove any lodged particles 98, a cleaning fluid is periodically passed through the analysis cone 96 in the opposite direction as the flow of the hand cleaning fluid 44 during the analysis. The cleaning fluid may, for example, be the same fluid as the hand cleaning fluid 44, or may optionally be a different fluid.

The analysis chambers 60 in the structures shown in Figures 5 to 7 are also preferably cleaned on a periodic basis, such as by passing a cleaning fluid through the chambers 60 in the same or opposite direction as the flow of the fluid 44 during the analysis. The cleaning preferably occurs automatically, without requiring maintenance staff. The cleaning may occur, for example, after a certain number of dispensing events, or after a certain amount of time has passed since the last cleaning.

It will be understood that, although various features of the invention have been described with respect to one or another of the embodiments of the invention, the various features and embodiments of the invention may be combined or used in conjunction with other features and embodiments of the invention as described and illustrated herein.

The invention is not limited to the particular structure of the fluid dispenser 10 shown in the drawings. Rather, any fluid dispenser 10 that is capable of analyzing the hand cleaning fluid 44 that has contacted a user's hand 52 so as to collect evidence of a possible infection could be used. Any suitable fluid dispenser 10 construction could be adapted to perform the methods of the present invention, including for example those taught in United States Patent No. 8,245,877 to Ophardt, issued August 21, 2012; United States Patent No. 8,113,388 to Ophardt et al., issued February 14, 2012; United States Patent No. 8,091,739 to Ophardt et al., issued January 10, 2012; United States Patent No. 7,748,573 to Anhuf et al., issued July 6, 2010; U.S. Patent No. 7,984,825 to Ophardt et al., issued July 26, 2011; U.S. Patent No. 8,684,236 to Ophardt, issued April 1, 2014; U.S. Patent No. 5,373,970 to Ophardt, issued December 20, 1994; U.S. Patent No. 5,836,482 to Ophardt et al., issued November 17, 1998; and U.S. Patent No. 9,682,390 to Ophardt et al., issued June 20, 2017, which are each incorporated herein by reference.

The invention is not limited to any particular fluid analysis mechanism 54. Rather, any suitable mechanism for analyzing the fluid 44 could be used, including mechanisms that use electrical, acoustic, optical, magnetic, spectroscopic, electromagnetic, mechanical, thermal, and/or chemical methods. A system in accordance with the invention could also include fluid dispensers 10 that have different fluid analysis mechanisms 54. The system could also include fluid dispensers 10 that have fluid analysis mechanisms 54, as well as fluid dispensers 10 that do not have fluid analysis mechanisms 54.

In at least some preferred embodiments of the invention, the analysis of the fluid 44 can be imperfect, and does not need to provide conclusive proof of the presence of an infection. Rather, the analysis is preferably low cost and fast, and can be used for widespread screening of a population or group of individuals to provide an indication of the presence of a possible infection or infections, so that rapid interventions can be implemented and further confirmatory investigations can take place.

When the systems and/or fluid dispensers 10 in accordance with the present invention are described herein as "detecting" an infection, it is to be appreciated that this refers to the systems and/or fluid dispensers 10 obtaining information or data that is interpreted by the systems and/or fluid dispensers 10 as indicating the presence of an infection. In some circumstances, the systems and/or fluid dispensers 10 may provide false positives, in which an infection is detected but no actual infection is present, as well as false negatives, in which no infection is detected but an actual infection is present. False positives and false negatives are a common occurrence for many diagnostic tests, and the present invention includes within its scope systems and/or fluid dispensers 10 that may provide false positives and/or false negatives under certain circumstances.

The present invention may be used to obtain a measure of the concentration of any particles 68 of interest in a fluid 44, and is not limited to the particular particles 68 identified in the preferred embodiments. For example, the particles 68 detected and measured could include one or more of the following: viral particles, bacterial particles, prions, parasites, pathogens, spores, and fungal particles. As used herein, the term "viral particles" includes live viruses, dead viruses, fractions of viruses, and clusters of viruses. The term "bacterial particles" as used herein includes live bacteria, dead bacteria, individual bacteria cells, clusters of bacteria cells, chains of bacteria cells, and fractions of bacteria cells.

The term "fluid" as used herein includes any flowable substance, including gases, liquids, solutions, foams, emulsions, acids, bases, and dispersions.

In embodiments of the present invention in which the fluid dispensers 10 are communicatively linked to a server 70, the fluid dispensers 10 are preferably configured to provide one or more of the following pieces of information to the server 70 when an infection is detected: the operating status of the fluid dispenser 10; the usage history of the dispenser 10; that an infection has been detected; the raw data from which the presence of an infection was inferred; a processed form of the data from which the presence of an infection was inferred; the location of the dispenser 10; the time when the infection was detected; the identity of the user that was using the dispenser 10 when the infection was detected; physiological data about the user; and the effectiveness of the hand cleaning/sanitizing event.

Preferably, each fluid dispenser 10 in the system is equipped with the necessary processing power and programming to independently analyze the data collected by the fluid analysis mechanism 54 so as to determine whether or not an infection is present, without requiring external input from the server 70. Each fluid dispenser 10 is furthermore preferably equipped with suitable software such as a machine learning algorithm or artificial intelligence that allows the fluid dispenser 10 to independently generate classification criteria for particles 68 using training data collected by the fluid dispenser 10 itself, and to classify detected particles 68 using the classification criteria. The fluid dispensers 10 are furthermore preferably able to independently update and improve the classification criteria and the thresholds and methods for detecting an infection using unsupervised machine learning. Having the fluid dispensers 10 operate independently as much as possible preferably allows the system to remain up to date and able to respond rapidly to new infections and situations, without requiring time consuming and possibly delayed instructions or updates from a central server 70.

Optionally, the parameters that are considered by the fluid dispenser 10 when determining the classification, such as particle size, shape, movement pattern, movement distance, and movement speed, are predetermined by an administrator or user of the system. The fluid dispenser 10 is preferably configured to independently define the values for each of the parameters that are used to define the various classes of particles 68. Any suitable parameters useful for classifying the particles 68 may be used, including for example area, circularity, electrical properties, body temperature, aspect ratio, density, solidity, perimeter, diameter, speed, speed standard deviation, speed linear regression, total angle, total angle standard deviation, angle linear regression, linear function error, linear function squared error, length, small axis (ellipse), large axis (ellipse), diffusion coefficient, anomalous exponent, asymmetry, efficiency, fractal dimension, gaussianity, kurtosis, mean standard displacement ratio, straightness, trappedness, X-coordinates of trajectory, Y-coordinates of trajectory, time between two points, and number of points. The system may also use one or more of the parameters disclosed in United States Patent Application Publication No. 2021/0123851 to Steltenkamp et al., published April 29, 2021, which is incorporated herein by reference.

In embodiments of the invention in which the system includes a server 70, the server 70 is also preferably equipped with the necessary processing power and programming to act quickly and independently with minimal input from a system administrator. The server 70, for example, is preferably provided with suitable software such as a machine learning algorithm or artificial intelligence that is able to independently interpret and respond to data received from the network of fluid dispensers 10, and to improve its performance over time. This preferably allows the system to respond rapidly to new situations and possible infections, without being delayed by human operators being required to review and analyze the incoming data.

In some preferred embodiments of the invention, the system may include an artificial intelligence classifier, which is tasked with selecting the parameter values that will define each of the classes; an artificial intelligence identifier, which is tasked with classifying particles 68 detected by the fluid analysis mechanism 54 into the different classes; and a master artificial intelligence, which is tasked with integrating all of the information received from the fluid dispensers 10 and external sources, and performing system level analysis and actions. Preferably, each fluid dispenser 10 is independently provided with its own artificial intelligence classifier and artificial intelligence identifier. The master artificial intelligence may be provided on the server 70 or servers 70.

Optionally, the classifier will set the parameters for defining the particles 68 using an n-dimensional phase space, where n is the number of parameters or so-called meta-parameters used. The number of (meta-)parameters defines the dimension of the phase space, and may for example vary from 3 up to more than 100. It is noteworthy that at the classification stage a particle 68 can be anything, ranging from a biological particle such as a pathogen, bacteria, or cell, such as a skin cell, or debris and clusters of biological particles, as well as any other kind of particles. The classifier may optionally do a pre-selection of particles 68 based on preliminary filters such as min-max area or any other relevant parameter in order to reduce the number of irrelevant particles 68.

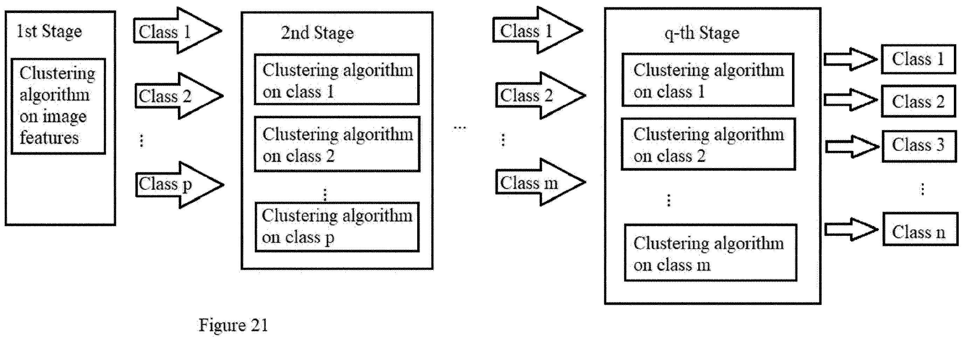

An example of a possible structure of the classifier is shown in Figure 21. In Figure 21, q is the depth of the classifier, which may for example be set by an administrator, user, or by the classifier itself. n > m > p.

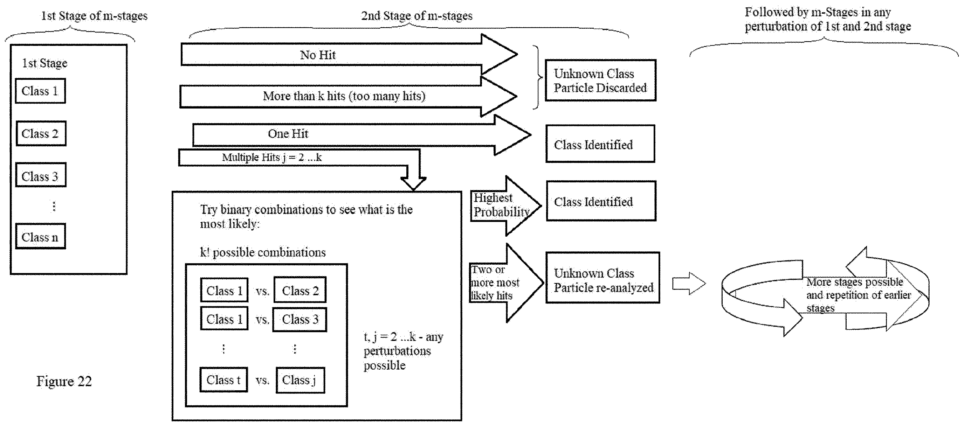

Optionally, the identifier uses a binary-architecture based on random forest algorithms. Other structures, such as neural networks, could be used as well. An example of a possible structure of the identifier is shown in Figure 22. In Figure 22, n = number of classes defined by the classifier; k = critical number of unknown hits (depth/accuracy of the identifier), which may for example be set by an administrator or user. k has to smaller than n. t, j = variables for the number of binary permutations for the next stage. m = number of stages, including the first classifier. It is also possible to replace the hits with a score table of characteristics, with the highest score corresponding to the most likely classes.

The inventors have found that allowing the classifier to select the parameters that define the classes of particles 68 can provide advantages in comparison to systems that are trained to identify specific species, such as E. coli. For example, systems that are trained to identify specific species require labor intensive training of the systems, whereas a system that selects classification parameters for itself can train itself when operating in real world conditions. Furthermore, a system that has been trained under laboratory conditions may have difficulty correctly classifying particles 68 under real world conditions. In addition, a system that has been trained to identify specific known species of bacteria may be unable to identify novel pathogens.

In some preferred embodiments of the invention, the system uses a stopped-flow analysis of the fluid 44. The stopped-flow analysis optionally uses a simple canal structure, which is cheap, user friendly, and not prone to becoming plugged or experiencing other errors. Thus, the system can preferably function for many months without requiring maintenance or service. The analysis channel or canal 60 may, for example, have a width of 5 to 10 mm, a length of 10 to 50 mm, and a height of 50 to 400 microns.

In embodiments of the invention that use an analysis cone 96, it is not necessary that the height of the internal cavity 102 decrease as the internal cavity 102 extends radially outwardly from the apex 98. Any suitable form of analysis chamber 60 could be used, and the invention is not limited to the particular examples shown.

Although the preferred embodiments have described the fluid analysis mechanism 54 as including a camera 66, this is not necessary. Rather, any suitable mechanism or mechanisms for analyzing the fluid 44 and detecting characteristics of the particles 68 could be used. For example, the fluid analysis mechanism 54 could include one or more detection devices or systems that use electrical, acoustic, optical, magnetic, spectroscopic, electromagnetic, mechanical, thermal, chemical, and/or any other suitable methods for analyzing the fluid 44. In some embodiments, the fluid analysis mechanism 54 may use any kind of transverse or longitudinal wave forms as well as any electrical detection methods, like impedance, pressure waves (longitudinal) or light waves (electromagnetic waves) and any combination thereof. Preferably, the fluid analysis mechanism 54 is capable of capturing data about the particles 68 that permit the particles 68 to be categorized into the recognized and unrecognized classes.

Although the preferred embodiments show the fluid collector 48 as having a plurality of apertures 46 through which the fluid 44 passes, this is not necessary. Rather, any suitable structure for collecting the fluid 44 could be used. Although the display 50 is shown at a particular location in the drawings, the display 50 could be placed at any suitable location, or could be omitted entirely.

In some embodiments of the invention, the particles 68 may be analyzed and/or categorized based at least in part on observed clustering of the particles 68. For example, the particles 68 could be analyzed and/or categorized based on the presence of clustering, the size of the clusters, the number of particles in each cluster, the number of direct and indirect neighbors in the clusters, the shape of clusters, and the angle of neighbors in the clusters. Different bacteria and/or particle types may exhibit different clustering characteristics, which can be used to categorize the particles into the recognized and unrecognized classes.

The invention optionally identifies the particles 68 as active or inactive based at least in part on the movement of the particles 68 over time, including the deceleration and stopping of inactive particles 68, and the continued movement of active or alive particles 68 over time. For example, various naturally occurring forces may cause dead or inactive particles 68 to stop moving over time, such as through sedimentation under the force of gravity, or the hydrodynamic resistance or drag force of the fluid 44. Other forces could also be applied to the fluid 44 in order to decelerate or stop the inactive particles 68, such as a stopping mechanism that uses magnetic force or optical traps. The active or alive particles 68 may continue to move despite the presence of these stopping forces, for example through the propulsive forces generated by their flagella.

Although this disclosure has described and illustrated certain preferred embodiments of the invention, it is to be understood that the invention is not restricted to these particular embodiments. Rather, the invention includes all embodiments which are functional, mechanical, chemical, electrical, or optical equivalents of the specific embodiments and features that have been described and illustrated herein.

## Claims

1. A system comprising:
a plurality of fluid dispensers (10) for dispensing hand cleaning fluid (44);
wherein at least some of the fluid dispensers (10) are configured to analyze at least some of the hand cleaning fluid (44) after the hand cleaning fluid (44) has contacted a user's hand (52); and
wherein the system is configured to detect an infection based at least in part on the analysis of the hand cleaning fluid (44).

2. The system according to claim 1, wherein the plurality of fluid dispensers (10) are located at a plurality of different locations within an environment;
wherein the environment has a plurality of different zones (78A, 78B, 78C, 78D, 78E);
wherein at least one of the fluid dispensers (10) is located in each of the zones (78A, 78B, 78C, 78D, 78E); and
wherein, when the system detects an infection, the system is configured to identify which of the zones (78A, 78B, 78C, 78D, 78E) the infection is located in based on the location of the fluid dispenser (10) where the infection was detected.

3. The system according to claim 2, wherein the system is configured to record infections detected by the system over time; and
wherein the system is configured to determine a path that the infections have spread through the zones (978A, 78B, 78C, 78D, 78E) of the environment over time based on the locations of the fluid dispensers (10) where the infections were detected over time.

4. The system according to claim 3, wherein the system is configured to predict where the infections will spread through the zones (78A, 78B, 78C, 78D, 78E) of the environment over time; and
wherein the prediction is based at least in part on one or more of:
the path that the system has determined that the infections have spread through the zones (78A, 78B, 78C, 78D, 78E) of the environment over time;
a previous path that the system has determined that a previous infection has spread through the zones (78A, 78B, 78C, 78D, 78E) of the environment over time;
historical data of how past infections have spread through the environment over time;
known patterns of movement of people within the environment; and
predicted patterns of movement of people within the environment.

5. The system according to any one of claims 1 to 5, wherein, when the system detects an infection, the system is configured to determine an identity of the user of the fluid dispenser (10) where the infection was detected; and
wherein the system is configured to at least one of:
identify the user based at least in part on facial recognition;
identify the user based at least in part on communications between the fluid dispenser (10) and a device carried by the user;
identify the user based at least in part on an analysis of a skin microbiome of the user;
notify a facility administrator of the identity of the user;
track movements of the user over time; and
notify a public health authority of the identity of the user.

6. The system according to any one of claims 1 to 5, wherein, when the system detects an infection, the system is configured to provide instructions to the user of the fluid dispenser (10) where the infection was detected to perform an action;
wherein the instructions are communicated to a communication device carried by the user; and
wherein the action comprises at least one of:
exiting a facility;
sanitizing the user's hands;
wearing a mask;
isolating;
seeking medical attention; and
performing a medical test.

7. The system according to any one of claims 2 to 4, wherein, when the system detects an infection, the system is configured to provide a notification in the zone (78A, 78B, 78C, 78D, 78E) where the infection was detected; and
wherein the notification comprises a visual and/or audible signal that is provided by at least one of the fluid dispensers (10) in the zone where the infection was detected.

8. The system according to claim 4, wherein, when the system detects an infection, the system is configured to provide a notification in one or more of the zones (78A, 78B, 78C, 78D, 78E) where the system predicts that the infection will spread; and
wherein the notification comprises a visual and/or audible signal that is provided by at least one of the fluid dispensers (10) in the one or more zones (78A, 78B, 78C, 78D, 78E) where the system predicts that the infection will spread.

9. The system according to any one of claims 1 to 8, wherein at least some of the fluid dispensers (10) have a fluid analysis mechanism (54);
wherein, in at least one mode of operation of the system, a subset of the fluid analysis mechanisms (54) are inactivated;
wherein, when the system detects an infection, the system is configured to modify an operation of at least one of the fluid dispensers (10); and
wherein the modification comprises at least one of:
providing a signal encouraging hand cleaning;
changing a formulation of the hand cleaning fluid (44);
changing a volume of the hand cleaning fluid (44) that is dispensed;
changing an operation of at least one of the fluid analysis mechanisms (54); and
activating at least one of the fluid analysis mechanisms (54) that were previously inactivated.

10. The system according to any one of claims 1 to 9, wherein at least some of the fluid dispensers (10) have a fluid analysis mechanism (54) for analyzing at least some of the hand cleaning fluid (44) after the hand cleaning fluid (44) has contacted the user's hand (52), the fluid analysis mechanism (54) comprising:
at least one detection device that captures data about particles (68) present in the hand cleaning fluid (44);
wherein the system is configured to categorize the particles (68) into predetermined classes based at least in part on the data;
wherein the predetermined classes comprise at least one recognized class and at least one unrecognized class;
wherein the system is configured to categorize the particles (68) that meet predetermined criteria into the at least one recognized class; and
wherein the system is configured to categorize the particles (68) that fail to meet the predetermined criteria into the at least one unrecognized class.

11. The system according to any one of claims 1 to 10, wherein at least some of the fluid dispensers (10) have a fluid analysis mechanism (54) for analyzing at least some of the hand cleaning fluid (44) after the hand cleaning fluid (44) has contacted the user's hand (52), the fluid analysis mechanism (54) comprising:
a fluid collector (48) for collecting at least some of the hand cleaning fluid (44) after the hand cleaning fluid (44) has contacted the user's hand (52);
an analysis chamber (60) that is fluidly connected to the fluid collector (48) for receiving at least some of the hand cleaning fluid (44) collected by the fluid collector (48); and
a camera (66) that captures at least one image of the hand cleaning fluid (44) received by the analysis chamber (60);
wherein the system is configured to detect particles (68) in the at least one image;
wherein the system is configured to categorize the particles (68) into predetermined classes;
wherein the predetermined classes comprise at least one recognized class and at least one unrecognized class;
wherein the system is configured to categorize the particles (68) that meet predetermined criteria into the at least one recognized class; and
wherein the system is configured to categorize the particles (68) that fail to meet the predetermined criteria into the at least one unrecognized class.

12. The system according to claim 11, wherein the system comprises a machine learning algorithm that is configured to select the predetermined criteria based on training data;
wherein the training data comprises images of the hand cleaning fluid (44) captured by the camera (66) during a training operation;
wherein the system is configured to determine a background rate of the particles (68) that are categorized into each of the at least one recognized class and each of the at least one unrecognized class based at least in part on images of the hand cleaning fluid (44) captured by the camera (66) over time;
wherein the system is configured to detect the infection based at least in part on detection of an increase in a quantity of the particles (68) that are categorized into at least one of the at least one recognized class and the at least one unrecognized class by at least a threshold amount above the background rate; and
wherein the predetermined criteria comprise at least one of:
a size of the particles (68);
a shape of the particles (68);
a movement of the particles (68); and
a cluster characteristic of the particles (68).

13. The system according to claim 11 or claim 12, wherein the camera (66) is configured to capture a series of images of the hand cleaning fluid (44) received by the analysis chamber (60);
wherein the fluid analysis mechanism (54) comprises a stop mechanism that stops a flow of the hand cleaning fluid (44) through the analysis chamber (60);
wherein at least some of the series of images are captured after the flow of the hand cleaning fluid (44) through the analysis chamber (60) has stopped;
wherein the system is configured to identify the particles (68) as active or inactive based at least in part on the movement of the particles (68) over time after the flow of the hand cleaning fluid (44) through the analysis chamber (60) has stopped;
wherein the system is configured to calculate a sanitation effectiveness value based at least in part on a comparison of a quantity of the particles (68) that are identified as active and a quantity of the particles (68) that are identified as inactive;
wherein the system is configured to modify an operation of the fluid dispenser (10) if the sanitation effectiveness value falls below a predetermined standard; and
wherein the modification comprises at least one of:
changing a formulation of the hand cleaning fluid (44) that is dispensed; and
changing a volume of the hand cleaning fluid (44) that is dispensed.

14. The system according to claim 13, wherein the analysis chamber (60) comprises an analysis cone (96);
wherein the analysis cone (96) has an apex (98), a base (100), and an internal cavity (102);
wherein the internal cavity (102) is in fluid communication with a fluid inlet (104) located at the apex (98);
wherein the internal cavity (102) is in fluid communication with a plurality of fluid outlets (106) located around a periphery of the base (100);
wherein a height of the internal cavity (102) decreases as the internal cavity (102) extends radially outwardly from the apex (98);
wherein the internal cavity (102) is defined between an upper surface (108) and a lower surface (110);
wherein a distance between the upper surface (108) and the lower surface (110) decreases as the internal cavity (102) extends radially outwardly from the apex (98); and
wherein the predetermined criteria comprise at least one of:
a location where the particles (98) become lodged between the upper surface (108) and the lower surface (110); and
a distance that the particles (98) drift after the flow is stopped.

15. The system according to claim 13 or claim 14, wherein the stop mechanism is configured to equalize a fluid pressure upstream of the analysis chamber (60) and downstream of the analysis chamber (60), so as to stop the flow of the hand cleaning fluid (44) through the analysis chamber (60);
wherein the stop mechanism comprises an upstream valve (88), a downstream valve (90), and an equalization conduit (92);
wherein the upstream valve (88) is located upstream of the analysis chamber (60);
wherein the downstream valve (90) is located downstream of the analysis chamber (60);
wherein the upstream valve (88) has a flow position, in which the analysis chamber (60) is fluidly disconnected from the equalization conduit (92) via the upstream valve (88);
wherein the downstream valve (90) has a flow position, in which the analysis chamber (60) is fluidly disconnected from the equalization conduit (92) via the downstream valve (90);
wherein the upstream valve (88) has a stop position, in which the analysis chamber (60) is in fluid communication with the equalization conduit (92) via the upstream valve (88); and
wherein the downstream valve (90) has a stop position, in which the analysis chamber (60) is in fluid communication with the equalization conduit (92) via the downstream valve (90).
